# EUROPEAN PATENT SPECIFICATION

(11) **EP 4 185 857 B1**
(45) Date of publication and mention of the grant of the patent: **25.06.2025**
(21) Application number: 21755280.1
(22) Date of filing: 23.07.2021
(51) Int. Cl.: G01N 21/64, G01N 21/27, G01J 3/44, C12Q 1/686

(54) **SYSTEMS AND METHODS FOR BIOLOGICAL ANALYSIS**
SYSTEME UND VERFAHREN ZUR BIOLOGISCHEN ANALYSE
SYSTÈMES ET MÉTHODES D'ANALYSE BIOLOGIQUE

(30) Priority: 23.07.2020 US 202063055459 P
(43) Date of publication of application: 31.05.2023
(73) Proprietor: Life Technologies Corporation, Carlsbad, CA 92008 (US)
(72) Inventor: FREUDENTHAL, Jacob, Carlsbad, 92008 (US); BENSON, Scott, Carlsbad, California 92008 (US)
(74) Representative: Hoffmann Eitle
(86) International application number: PCT/US2021/042997
(87) International publication number: WO 2022/020729

(56) References cited:
- CN-B- 101 189 505
- US-A1- 2002 034 746
- US-A1- 2004 014 202
- US-A1- 2005 036 142
- US-A1- 2006 138 344
- US-A1- 2016 230 213
- US-A1- 2018 157 023
- US-A1- 2018 201 987

## Description

### Cross Reference to Related Applications

This application claims priority from U.S. Provisional Application No. 63/055,459, filed July 23, 2020.

### Technical Field

The present disclosure relates generally to systems, and methods for observing, testing, and/or analyzing one or more biological samples, and more specifically to systems, and methods comprising optical systems for observing, testing, and/or analyzing one or more biological samples. The present disclosure further relates to systems, and methods for observing, testing, and/or analyzing one or more biological samples by quantitative polymerase chain reaction (qPCR) or digital PCR (dPCR), and more specifically to systems, and methods comprising optical systems for observing, testing, and/or analyzing one or more biological samples by qPCR or dPCR using the energy transfer dye conjugate pairs.

### Background

There are multiple modes of energy transfer (ET) including Dexter energy transfer and Förster energy transfer. Energy transfer can involve fluorescence quenching mechanisms whereby an excitation electron can be transferred from a donor molecule to an acceptor molecule via a non-radiative path. Förster energy transfer can occur when there is interaction between the donor and acceptor. In certain embodiments, the donor dye is a rhodamine or a cyanine dye, and the reporter dye is a cyanine dye (e.g., an azaindoline cyanine) that emits in the far-red or near-IR region of the spectrum.

Real-time systems for quantitative PCR (qPCR) were improved by probe-based, rather than intercalator-based PCR product detection. Real-time systems for quantitative PCR (qPCR) are frequently used to conduct assays on cell and tissue samples. One probe-based method for detection of amplification products without separation from the primers is the 5' nuclease PCR assay (also referred to as the TaqMan^{®} probe (Roche Molecular Systems) assay or hydrolysis probe assay). This alternative method provides a real-time method for detecting only specific amplification products. During amplification, annealing of the hydrolysis probe, sometimes referred to as a "TaqMan probe", to its target sequence generates a substrate that is cleaved by the 5' nuclease activity of a DNA polymerase, such as a Taq DNA polymerase, when the enzyme extends from an upstream primer into the region of the probe. This dependence on polymerization ensures that cleavage of the probe occurs only if the target sequence is being amplified.

Current analyses of cell and tissue functionality often require extracting as much information as possible from materials that are often limited. For example, samples such as tumor biopsies are difficult to collect and usually yield only a small amount of usable nucleic acid. PCR detection and measurement of a single target analyte, referred to as a single-plex assay, has been the gold standard for analyzing clinical research samples on the nucleic acid level, and has been invaluable in extending the limits of biological knowledge for more than a quarter century.

However, the limited amount of nucleic acid obtained from clinical research specimens often forces choices to be made about how best to utilize these precious samples. Furthermore, if the sample is limited, the number of loci that can be analyzed is also limited, reducing the amount of information that can be extracted from a single sample. Finally, the additional time and materials required to set up multiple single-assay reactions could increase the expense of a complex project significantly.

Real-time systems for quantitative PCR (qPCR) are frequently used to conduct assays on cell and tissue samples. Nucleic acid detection/amplification methods, such as in real-time polymerase chain reactions, frequently use dual-labeled probes to detect and/or quantify target nucleic acids like specific gene sequences or expressed messenger RNA sequences. Fluorogenic probes for use in such methods are often labeled with both a reporter and a quencher moiety. In such cases, fluorescence from the reporter is unquenched when the two moieties are physically separated via hybridization of the oligonucleotide probe to a nucleic acid template and/or via nuclease activity which removes one of the quencher or reporter moieties components from the oligonucleotide probe.

Fluorescence resonance energy transfer (FRET) within dual-labeled oligonucleotide probes is widely used in assays for genetic analysis. FRET has been utilized to study DNA hybridization and amplification, the dynamics of protein folding, proteolytic degradation, and interactions between other biomolecules. FRET can occur between reporter and quencher groups and can involve different modes of energy transfer (ET). For example, energy transfer can involve fluorescence quenching mechanisms whereby an excitation electron can be transferred from a donor molecule to an acceptor molecule via a non-radiative path when there is interaction between the donor and acceptor. FRET also can occur between two dye molecules when excitation is transferred from a donor molecule to an acceptor molecule without emission of a photon.

Multiplex PCR analysis of nucleic acids, a strategy where more than one target is amplified and quantified from a single sample aliquot, is an attractive solution to problems associated with running multiple single-plex assays. In multiplex PCR, a sample aliquot is queried with multiple probes that contain fluorescent dyes in a single PCR reaction. This increases the amount of information that can be extracted from that sample. With multiplex PCR, significant savings in sample and materials can be realized. To increase the utility of this method, multiplexed PCR using several pairs of gene-specific primers and probes to amplify and measure multiple target sequences simultaneously have been developed. Multiplexing PCR provides the following advantages: 1) Efficiency: multiplexed PCR helps conserve sample material and avoid well-to-well variation by combining several PCR assays into a single reaction. Multiplexing makes more efficient use of limited samples, such as those harboring a rare target that cannot be split into multiple aliquots without compromising the sensitivity; 2) Economy: even though the targets are amplified in unison, each one is detected independently by using a gene-specific probe with a unique reporter dye to distinguish the amplifications based on their fluorescent signal. Once optimized, a multiplexed assay is more cost effective than the same assays amplified independently.

However, currently there are limitations to the number of targets that can be analyzed in a single multiplex PCR assay. The experimental design for multiplex PCR is more complicated than for single reactions. The probes used to detect individual targets must contain unique reporter moieties with distinct spectra. The settings for excitation and emission filters of real-time detection systems vary from manufacturer to manufacturer; therefore, instruments must be calibrated for each dye moiety as part of the experiment optimization process. Thus, one limitation in the development of multiplex PCR assays is the number of fluorophores, and hence probes, that can be effectively measured in a single reaction Another limitation in multiplexed PCR results from signal interference ("cross-talk") between different fluorescence reporters that can compromise quantification or cause false positives or inaccurate quantification. Using traditional systems, it is therefore important to select fluorophores with minimal spectral overlap. Thus, when designing multiplexed reactions, different targets should be labeled with fluorophores that avoid overlapping excitation and/or emission profiles to avoid possible crosstalk issues. Additionally, the emission and excitation spectra of the fluorophores must be compatible with the PCR instrument to be used, and specifically, the band-pass specifications for each filter-set.

In addition, when designing multiplexed reactions, it is desirable to avoid possible cross-talk between fluorescence reporters. Signal cross-talk can also be minimized by designing fluorescent probes that quench well. Efficient quenching can be achieved by ensuring that the reporter and quencher moieties are compatible. An example of a compatible dye/quencher combination for a TaqMan probe is a FAM fluorophore with a TAMRA quencher. More recently, however, "dark quenchers", such as Dabcyl and Black Hole Quenchers (BHQ), have largely replaced fluorescent quenchers such as TAMRA. Dark quenchers emit the energy they absorb from the fluorophore as heat rather than light of a different wavelength. "Dark quenchers" tend to give results with lower background, and are especially useful in a multiplex reaction where it is important to avoid emitted light from the quencher creating cross-talk signal with one of the reporter dyes. Thus, highly efficient "dark quenchers" considerably reduce background fluorescence from fluorophore and quencher moieties leading to increased sensitivity and end-point signal. This is particularly useful for multiplex reactions because having several fluorophores in the same tube causes higher background fluorescence. Also provided herein is an oligonucleotide probe coupled to an ET conjugate, as disclosed herein, that is further coupled to a quencher, wherein the quencher is a dibenzoxanthene compound. In certain embodiments, the dibenzoxanthene compound is an imino-dibenzoxanthene compound, such as a substituted 3-imino-3H-dibenzo[c,h]xanthen-11-amine compound.

In general, multiplex PCR reactions have been limited due, for example, to complexities in the chemistry introduced when a large number of different probes are present within a single reaction mixture. Currently used probe combinations include: duplex reactions using dyes such as FAM and HEX (JOE/VIC^{®}); triplex reactions using dyes such as FAM, HEX (JOE/VIC^{®}), NED or Cy5; and quadriplex reactions using dyes such as FAM, HEX (JOE/VIC^{®}), Texas Red^{®}, and Cy5 dyes. Until recently, the most common multiplex PCR instruments could take advantage of only four unique dye-quencher pairs.

Chemical complexities notwithstanding, higher-plex qPCR assays have also been limited by instrument capabilities or the way in which existing instruments are utilized. Currently available qPCR instruments divide a broad excitation and emission spectrum into distinct spectra (EX/EM "channels") that are compatible with the excitation/emission spectra of corresponding sets of dyes or probes. For example, matched sets of EX/EM channels have typically been used up to accommodate quadriplex reactions, as discussed above. However, due at least in part to the unavailability of suitable probes, a fuller range of EX/EM channel combinations have yet to be utilized to provide assays able to quantify more than 4 targets in a common sample.

Thus, there is a need to provide additional probes that include unique fluorophore/quencher combinations that allow for increased multiplex reactions and detection through the additional spectral channels already available on some commercial instruments. Further, there is a need for new fluorophores and fluorophore/quencher combinations with unique optical properties that can facilitate even higher order multiplexing once instruments with additional channels and other related hardware and software improvements become available. There is also a need to develop new instruments and/or configure existing instruments to utilize these additional probes in support of higher levels of multiplexing, e.g., 6-plex PCR, 8-plex PCR, 10-plex PCR, 20-plex PCR, and the like.

US 2006/138344 A1 discloses a system for illuminating a sample, the system comprising a light source emitting light in the visible range, an excitation filter, a plurality of fluorochrome dyes for the sample, a detector configured to measure emissions from the sample, first and second emission filters respectively corresponding to the dyes, and a processor comprising at least one memory for real-time PCR processing of the detected emission light.

### Summary

The present disclosure provides systems and a method as set out in the appended set of claims.

Additional embodiments, features, and advantages of the disclosure will be apparent from the following detailed description and through practice of the disclosure. It will be understood that any of the embodiments described herein can be used in connection with any other embodiments described herein to the extent that the embodiments do not contradict one another.

### Brief Description of the Drawings

Embodiments of the present disclosure may be better understood from the following detailed description when read in conjunction with the accompanying drawings. Such embodiments, which are for illustrative purposes only, depict novel and non-obvious aspects of the present disclosure. The drawings include the following figures:
FIG. 1 is a schematic representation of a system according to an embodiment of the present disclosure.
FIG. 2 is a schematic representation of another system according to an embodiment of the present disclosure.
FIG. 3 is a schematic representation of another system according to an embodiment of the present disclosure.
FIG. 4 is a perspective view of a system according to an embodiment of the present disclosure.
FIG. 5 is a schematic representation of system according to an embodiment of the present disclosure that may include any of the systems illustrated in FIGS. 1-4.
FIG. 6 is a tabular representation of a set of ex-em channels and dyes according to an embodiment of the present disclosure.
FIG. 7 is a method according to an embodiment of the present disclosure.
FIG. 8 is an absorption or excitation spectrum and associated emission spectrum for a pair of dyes according to an embodiment of the present disclosure.
FIG. 9 is a tabular representation of a set of ex-em channels and dyes according to an embodiment of the present disclosure including the dyes shown in FIG. 8.
FIG. 10 is a graph of an ex-em channel space of the dyes shown in FIG. 8.
FIG. 11 is a method according to an embodiment of the present disclosure.
FIG. 12 is an absorption or excitation spectrum and associated emission spectrum for three dyes according to an embodiment of the present disclosure.
FIG. 13 is a tabular representation of a set of ex-em channels and dyes according to an embodiment of the present disclosure including the dyes shown in FIG. 12.
FIG. 14 is a graph of an ex-em channel space of the dyes shown in FIG. 12.
FIG. 15 is an absorption or excitation spectrum and associated emission spectrum for three dyes according to an embodiment of the present disclosure.
FIG. 16 is an absorption or excitation spectrum and associated emission spectrum for five dyes according to an embodiment of the present disclosure.
FIG. 17 is a tabular representation of a set of ex-em channels and dyes according to an embodiment of the present disclosure including the dyes shown in FIG. 16.
FIG. 18 is a graph of an ex-em channel space of the dyes shown in FIG. 16.
FIG. 19 is a tabular representation of a set of ex-em channels and dyes according to an embodiment of the present disclosure including a set of 10 dyes.
FIG. 20 is a method according to an embodiment of the present disclosure.

### Detailed Description of the Drawings

Reference will now be made in detail to certain embodiments of the disclosure, which are generally directed to systems, devices, and methods for preparing, observing, testing, and/or analyzing one or more biological samples. The embodiments discussed herein are generally directed to amplification devices, systems, and methods such as polymerase chain reaction (PCR) devices, systems, and methods, for example, real-time PCR (qPCR) devices, systems, and methods or end-point devices, systems, and methods such as digital PCR (dPCR) or melt curve analysis devices, system, or methods. Such description is not intended to limit the scope of the present disclosure, but merely to provide a description of embodiments.

As used herein, the terms "wavelength range", "wavelength band", or the like, may mean a "full width at half maximum" (FWHM) wavelength range or wavelength band. As used herein, the terms FWHM wavelength range or FWHM wavelength band means a wavelength range or wavelength band having an extent equal to a difference between maximum and minimum wavelength values at which the radiation through, from, or off an optical element (e.g., a source of radiation or a spectral filter, mirror, beamsplitter, grating, or the like) is equal to one half a maximum value within the wavelength range or wavelength band of that element.

For a spectrum defined by a spectral function (e.g., an absorption, excitation, or emission spectral function) over a wavelength range, as used herein, a "peak wavelength" or "maximum wavelength" (e.g., "maximum absorption wavelength", "maximum excitation wavelength", "maximum emission wavelength") means a wavelength at which the spectrum function decreases as the wavelength increases or decreases about the maximum or peak wavelength (e.g., the absorption, excitation, or emission decreases as the wavelength increases or decreases from the maximum or peak wavelength - for example, increases or decreases by 2 nanometers). As used herein, the peak or maximum wavelength may be a local peak or maximum over a predetermined portion of a total spectrum or may be an "absolute peak" or "absolute maximum" in which the value of the spectral function is greater than at any other wavelength over an entirety of the spectrum.

As used herein, the peaks or maximums of two or more spectra (e.g., an absorption, excitation, or emission spectra of two or more dyes) may be considered "equal", "near", or "substantially equal" to one another when the difference between the peaks or maximums of two or more spectra is less than or equal to 15 nanometers. As used herein, a peak or maximum of a spectrum (e.g., an absorption, excitation, or emission spectrum of a dye) may be considered "near" a referenced wavelength band (e.g., of an excitation or emission channel, spectral element, or filter) when the peak or maximum of the spectrum is within 15 nanometers of a minimum or maximum limit of the referenced wavelength band. As used herein, a peak or maximum of a spectrum (e.g., an absorption, excitation, or emission spectrum of a dye) may be considered "nearest" or "closest" to a referenced wavelength band belonging to a set of available wavelength bands (e.g., the wavelength bands of a set of excitation or emission channels, spectral elements, or filters) when the integrated value of the spectrum over the referenced wavelength band is greater than the integrated value of the spectrum over any of other wavelength band of the set of available wavelength bands.

As used herein a "radiant generator" or "generator" means a source capable of producing electromagnetic radiation. The radiant generator may comprise a single source of light, for example, an incandescent lamp, a gas discharge lamp (e.g., Halogen lamp, Xenon lamp, Argon lamp, Krypton lamp, etc.), a light emitting diode (LED), a white light LED, an organic LED (OLED), a laser (e.g., chemical laser, excimer laser, semiconductor laser, solid state laser, Helium Neon laser, Argon laser, dye laser, diode laser, diode pumped laser, fiber laser, pulsed laser, continuous laser), or the like. Alternatively, the radiant generator may comprise a plurality of individual radiant generators (e.g., a plurality of LEDs or lasers) each configured to produce a different wavelength range or band that may be non-overlapping or partially overlapping with the other individual radiant generators. The radiant generator may be characterized by electromagnetic radiation that is primarily within the visible light range (e.g., a "light source" emitting electromagnetic radiation within a wavelength in the range of 400 nanometers to 700 nanometers or in the range of 380 nanometers and 800 nanometers), near infrared range, infrared range, ultraviolet range, or other ranges within the electromagnetic spectrum. The radiant generator may provide continuous or pulsed illumination, and may comprise either a single beam or a plurality of beams that are spatially and/or temporally separated.

As used herein, a "sample" refers to any substance containing, or presumed to contain, one or more biomolecules (e.g., one or more nucleic acid and/or protein target molecules) and can include one or more of cells, a tissue or a fluid isolated from an individual or individuals. Samples may be derived from a mammalian or non-mammalian organism (e.g., including but not limited to a plant, virus, bacteriophage, bacteria, and/or fungus). As used herein, the sample may refer to the substance contained in an individual solution, container, vial, and/or reaction site or may refer to the substance that is partitioned between an array of solutions, containers, vials, and/or reaction sites (e.g., substance partitioned over an array of microtiter plate vials or over an array of array of through-holes or reaction regions of a sample plate; for example, for use in a dPCR assay). In some embodiments, a sample may be a crude sample. For example, the sample may be a crude biological sample that has not undergone any additional sample preparation or isolation. In some embodiments, the sample may be a processed sample that had undergone additional processing steps to further isolate the analyte(s) of interest and/or clean up other debris or contaminants from the sample.

As used herein, the terms "target", "target molecule", "target biomolecule", "target analyte", "target sequence", "target nucleic acid molecule", or the like, refers to a molecule having a particular chemical structure that is distinct from that of one or more other "targets", "target molecules", "target biomolecules", "target analytes", "target sequences", "target nucleic acid molecules", or the like. In general, each "target", "target molecule", "target biomolecule", "target analyte", "target sequence", "target nucleic acid molecule", or the like contains a structure or sequence that is distinct from any structure or sequence of the others and that may be utilized to attached to a particular probe or dye contained in sample or sample solution.

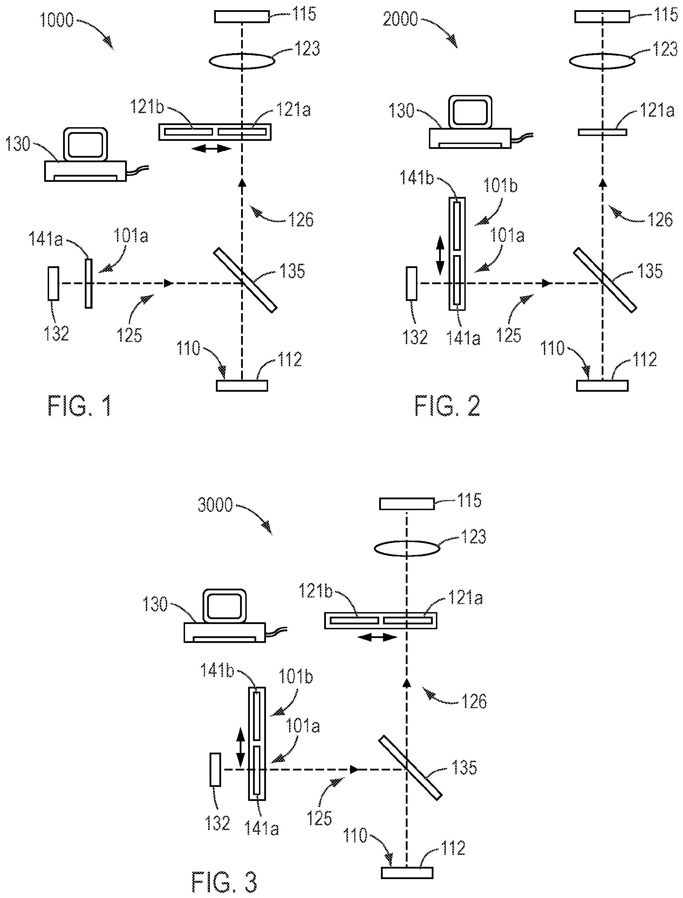

Referring to FIG. 1, in certain embodiments a system 1000 comprises a first radiant source 101a that is configured to illuminate a nucleic acid sample 110 located in or on a sample holder or container 112, the sample 110 being disposed to receive radiation from a first radiant source 101a. Sample 110 comprises a first dye that may be configured to bind to a first target molecule and a second dye that may be configured to bind to a second target molecule. Radiant source 101a is configured to produce excitation beams that are directed along an excitation optical path 125 to sample 110. System 1000 further comprises a sensor or detector 115 configured to measure emissions from sample 110, a first emission spectral element 121a characterized by a first average emission wavelength, and a second emission spectral element 121b characterized by a second average emission wavelength that is different than first average emission wavelength. An optical element or lens 123 may be used in combination with detector 115, for example, to reimage sample holder 112, sample 110, and/or emissions therefrom. Optical element 123 may be in the form of a transmissive and/or reflected optical element configured to focus or image light or radiation from sample holder 112 and/or sample 110.

Each emission spectral element 121a, 121b may be further characterized by wavelength band or range. The wavelength band or range of emission spectral elements 121a, 121b may be selected so that the wavelength band or range of emission spectral elements 121a does not overlap, or only partially overlaps, that of emission spectral element 121b. When the first target molecule and/or second target molecules are present in sample 110, emissions from corresponding first and/or second dyes are directed from sample 110 to detector 115 along an emission optical path 126. Emission spectral elements 121a, 121b may comprise respective filters passing or reflecting a wavelength band suitable for detecting and/or measuring a respective dye.

System 1000 may be configured to perform an amplification assay on sample 110. Optionally, the amplification assay may be performed on a separate system and further processed and/or examined using system 1000. System 1000 may also comprise at least one computer or processor 130 comprising at least one memory (not shown) including instructions to perform an amplification assay on sample 110. During and/or after the amplification assay, system 1000 is configured to illuminate sample 110 using radiant source 101a and to detect and/or measure emissions from any target molecules present using detector 115 in combination with emission spectral elements 121a, 121b. The memory associated with processor 130 may include instructions to, at least once during or after an amplification assay, illuminate sample 110 with first radiant source 101a and, in response, (1) to measure emissions from sample 110 using detector 115 and emission spectral element 121a and (2) to measure emissions from sample 110 using detector 115 and second emission spectral element 121b. As discussed in further detail below, the memory may comprise instructions to determine an amount of any target molecule present in sample 110, for example, by correlating measured emissions from sample 110 using first and second emission spectral elements 121a, 121b with an amount of the first and second target molecules, respectively.

Referring to FIG. 2, in another embodiment, a system 2000 comprises first radiant source 101a and a second radiant source 102, wherein first radiant source 101a is characterized by a first average excitation wavelength and second radiant source 102 is characterized by a second average excitation wavelength that is different than the first average excitation wavelength. Radiant sources 101a, 102 for systems 1000, 2000 may each be further characterized by wavelength band or range. The wavelength band or range of radiant sources 101a, 101b may be selected so that the wavelength band or range of radiant sources 101a does not overlap that of radiant sources 101b. Sample 110 comprises a first dye that may be configured to bind to a first target molecule and a second dye that may be configured to bind to a second target molecule, where either or both dyes may be different than the first and second dyes discussed above regarding system 1000. System 2000 may optionally include first emission spectral element 121a, which may be the same as or different from first emission spectral element 121a used in system 1000. Radiant sources 101a, 101b of systems 1000, 2000 may comprise a radiant emitter or radiant generator 132 in combination of a respective excitation spectral element 141a, 141b, which may, for example, be respective excitation filters passing or reflecting a wavelength band suitable for exciting a respective dye.

System 2000 may be configured to perform an amplification assay on sample 110. Optionally, the amplification assay may be performed on a separate system and subsequently processed and/or examined using system 2000. During and/or after the amplification assay, system 2000 is configured to illuminate sample 110 using radiant sources 101a, 101b and to detect and/or measure emissions from any target molecules present using detector 115 in combination with first emission spectral element 121a, when present. The memory associated with processor 130 in system 2000 may include instructions to perform an amplification assay on sample 110. The memory associated with processor 130 may include instructions to, at least once during or after an amplification assay, (1) illuminate sample 110 with first radiant source 101a and, in response, measure emissions from sample 110 using detector 115 and, optionally, first emission spectral element 121a and (2) illuminate sample 110 with second radiant source 101b and, in response, measure emissions from sample 110 using detector 115 and, optionally, first emission spectral element 121a. As discussed in further detail below, the memory may further comprise instructions to determine an amount of the first target molecule and/or an amount of the second target molecule when present in sample 110, for example, by correlating measured emissions from sample 110 when illuminating with first and second radiant sources 101a, 101b with an amount of the first and second target molecules, respectively. Where applicable, system 2000 may incorporate any of the elements or features discussed above herein regarding system 1000. Where applicable, processor 130 and associated memory of system 2000 may incorporate any of the elements or features of the processor 130 discussed above herein regarding system 1000.

With further reference to FIG. 3, in some embodiments, a system 3000 comprises both the first and second radiant sources 101a, 101b and both first and second emission spectral elements 121a, 121b. In such embodiments, sample 110 comprises a first dye that may be configured to bind to a first target molecule, a second dye that may be configured to bind to a second target molecule, and a third dye configure to bind to a third target molecule. As discussed above, first radiant source 101a is characterized by a first average excitation wavelength and second radiant source 101b is characterized by a second average excitation wavelength that is different than the first average excitation wavelength, while first emission spectral element 121a is characterized by a first average emission wavelength and second emission spectral element 121b is characterized by a second average emission wavelength that is different than the first average emission wavelength.

System 3000 may be configured to perform an amplification assay on sample 110. Optionally, the amplification assay may be performed on a separate system and further processed and/or examined using system 3000. During and/or after the amplification assay, system 1000 is configured to illuminate sample 110 using radiant sources 101a, 101b and to detect and/or measure emissions from any target molecules present using detector 115 in combination with emission spectral elements 121a, 121b. System 3000 may be configured to perform an amplification assay on sample 110. Optionally, the amplification assay may be performed on a separate system and further processed and/or examined using system 3000. During and/or after the amplification assay, system 3000 is configured to, at least once during or after the amplification assay, (1) illuminate sample 110 at least once with first radiant source 101a and, in response, detect or measure emissions from sample 110 using detector 115 and first emission spectral element 121a and detect or measure emissions from sample 110 using detector 115 and second emission spectral element 121b and (2) illuminate sample 110 with second radiant source 101b and, in response, detect or measure emissions from sample 110 using detector 115 and second emission spectral element 121b. As discussed in further detail below, the memory may comprise instructions to determine an amount of the first target molecule, the second target molecule, and/or the third target molecule when present in sample 110, for example, by correlating measured emissions from sample 110 when illuminating with first or second radiant sources 101a, 101b with an amount of the first, second, and third target molecules, respectively.

Where applicable, system 3000 may incorporate any of the elements or features discussed above herein regarding systems 1000, 2000. Where applicable, processor 130 of system 3000 may incorporate any of the elements or features of the processor 130 and associated memory discussed above herein regarding systems 1000, 2000. The embodiments of spectral elements 121a, 121b, 141a, 141b and sources 101a, 101b discussed above in relation to FIG. 1 and FIG. 2 may also apply to those elements and sources of system 3000. Any of systems 1000, 2000, or 3000 may include a filter wheel, translation stage, or the like, for example, to selectively move excitation spectral elements 141a, 141b into and out of excitation optical path 125. Any of systems 1000, 2000, or 3000 may include filter wheel, translation stage, or the like, for example, to selectively move emission spectral elements 121a, 121b into and out of emission optical path 126.

In certain embodiments, any of systems 1000, 2000, or 3000 may comprise at least one beam steering optical element 135 that steers or fold radiation from radiant sources 101a, 101b to sample 110. Alternatively, any of systems 1000, 2000, 3000 may be configured so that emissions form sample 110 are steered or directed to detector 115 using at least one beam steering optical element 135. Using that beam steering optical element 135, excitation and emission optical paths 125, 126 may comprise a common path portion where optical paths 125, 126 overlap from beam steering optical element 135 to sample 110. In other embodiments, excitation and emission optical paths 125, 126 do not overlap or have a common path portion except at sample 110. For example, emission beam optical path 126 may disposed normal or perpendicular to surface of sample holder 112, while excitation optical path 125 may be disposed at an off-axis angle from the surface that is not normal or perpendicular to the surface of sample holder 112. Systems 1000, 2000, 3000 may additionally or alternatively incorporate other optical configurations known in the art. For example, rather than in the reflective arrangement shown in FIG. 1-3, in which excitation and emission optical paths 125,126 are located on a common side or surface of sample holder 112, excitation and emission optical paths 125,126 may be configured in a transmissive arrangement in which the excitation optical path 125 is located on one side or surface of sample holder 112 and emission optical path 126 is located on an opposite side or surface of sample holder 112. Systems 1000, 2000, or 3000 may further comprise other optical elements for conditioning radiation or light from radiant sources 101a, 101b and/or emissions from sample 112.

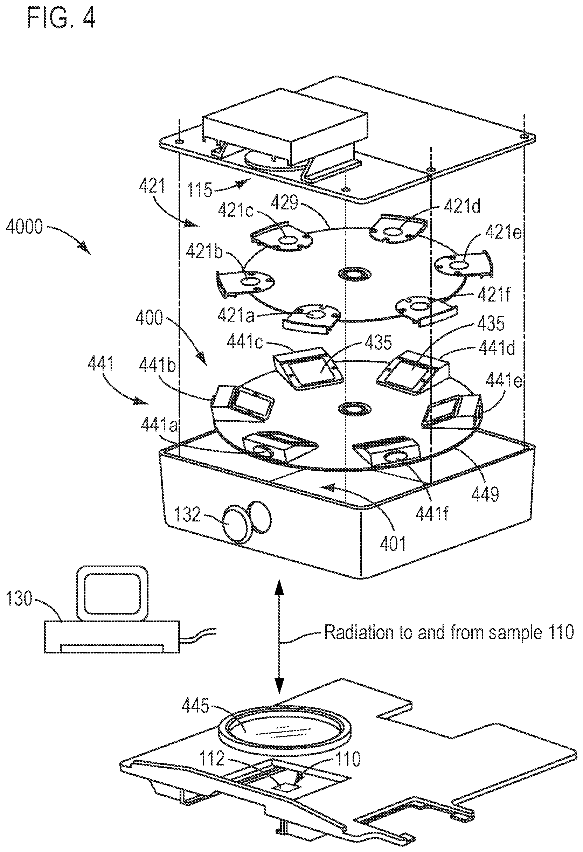

Referring to FIG. 4, in certain embodiments, a system 4000 comprises a plurality of radiant sources 401 and plurality of emission spectral elements 441 (441a-441f in the illustrated embodiment). In the illustrated embodiment, each radiant source 401 comprise radiant generator 132 and one of the six excitation spectral elements 441. In certain embodiments, each radiant source 401 may be characterized by a respective average excitation wavelength, wherein one or more of the six average excitation wavelengths is different from the remaining average excitation wavelengths. Each radiant source 401 may be characterized by a respective excitation wavelength band or range, wherein at least some of the excitation wavelength bands or ranges do not overlap the excitation wavelength band or range of any of the remaining radiant sources 401. In certain embodiments, any of radiant sources 401a-f may comprise one or more radiant generators 132 in combination with an excitation spectral element 401 (e.g., with a respective one of excitation spectral elements 401a-f).

System 4000 further comprises detector 115 and a plurality of emission spectral elements 421 (421a-421f in the illustrated embodiment) for use in combination with radiant sources 421a-f. Sample 110 may comprise three dyes associated with three target molecules, as discussed regarding system 3000. Because of the additional number radiant sources 401 and emission spectral elements 421, system 4000 is configured to detect and/or measure more than three dyes. Using six radiant sources 401 and six associated emission spectral elements 421, prior art systems are only able to detect and deconvolve at most six dyes to determine amounts of at most six corresponding target molecules. As discussed in further detail below herein, system 4000 is configured to detect and deconvolve more than six dyes so as to determine amounts of more than six corresponding target molecules. In certain embodiments, system 4000 may include a field lens 445 or other optical elements suitable for conditioning radiation from radiant generator 132 and/or emissions from sample 112.

While FIG. 4 illustrates a system 4000 comprising six radiant sources 401a-f and six emission spectral elements 421a-f. It will be appreciated that system 4000 may contain fewer than six of radiant sources and/or fewer than six emission spectral elements (e.g., like systems 1000, 2000, or 3000). In certain embodiments, system 4000 may contain more than six radiant sources and/or more than six emission spectral elements, for example, to increase the number of target molecules that may be detected or measured by system 4000 and/or to increase the accuracy or sensitivity of system 4000 to detect or measure a selected number of target molecules. For example, system 4000 may comprise seven, eight, or more radiant sources 401 and/or seven, eight, or more emission spectral elements 421. In certain embodiments, each emission spectral element 421 comprises a specific wavelength band or range from a chromatically dispersive optical element such as a prism, diffractive optical element, spectrometer, or spectrophotometer that is illuminated by emissions from sample 110. Additionally or alternatively, each excitation spectral element 441 comprises a specific wavelength band or range from a chromatically dispersive optical element such as a prism or diffractive optical element from the spectrum provided by one or more radiant generators 132.

Where applicable, the elements or components of system 4000 may take the form of embodiments of any corresponding element discussed above regarding any of systems 1000, 2000, 3000. For example, the radiant generator 132, sample 110, sample holder 112, and processor(s) 130 and associated memory(ies) may take the form of those same elements or components discussed above with regard to any of systems 1000, 2000, 3000. Any or all of excitation spectral elements 441 may take the form of any of the embodiments discussed above regarding excitation spectral element 141a or excitation spectral element 141b. Any or all emission spectral elements 421 may take the form of any of the embodiments discussed above regarding emission spectral element 121a or excitation spectral element 121b. Any or all radiant sources 401a-f may take the form of any of the embodiments discussed above regarding radiant source 101a or radiant source 101b.

Systems 1000, 2000, 3000, 4000 may incorporate other lenses, filters, mirror, prisms, diffractive optical element, or the like, that known in the biological instruments and systems art. In certain embodiments, further lenses or other imaging optical elements may be included along the excitation and emission optical paths of systems 1000, 2000, 3000, 4000 (e.g., optical paths 125, 126). For example, a field lens may be located proximal sample holder 112 (e.g., field lens 445 shown proximal sample holder 112 in FIG. 4). In some embodiments, further imaging optics are located along the optical path between the sample holder 112 and detector 115 to relay an image or otherwise condition emissions from sample 110. Embodiments of 1000, 2000, 3000, 4000 may incorporate any of the optical elements or configurations disclosed in US6818437, US2004/0009586, US9702823, or US2016/0230210.

Optical element 123 or any other imaging optical elements along the excitation and/or emission optical paths of systems 1000, 2000, 3000, 4000 may comprise one or more of a refractive lens, a mirror, a diffractive or holographic optical element, or the like. One or more of spectral elements 121, 141, 421, 441 may comprise one or more of a colored substrate, a dichroic element such as a filter, mirror, or beamsplitter, or a dispersive optical element such as a prism, diffractive grating, or holographic grating. For any of the systems 1000, 2000, 3000, 4000, detector 115 may comprise may comprise one or more individual photodetectors including, but not limited to, photodiodes, photomultiplier tubes, bolometers, cryogenic detectors, quantum dots, light emitting diodes (LEDs), semiconductor detectors, HgCdTe detectors, or the like. Additionally or alternatively, detector 115 may comprise an array sensor including an array of sensors or pixels. The array sensor may comprise one or more of a complementary metal-oxide-semiconductor sensor (CMOS), a charge-coupled device (CCD) sensor, a plurality of photodiodes detectors, a plurality of photomultiplier tubes, or the like. In certain embodiments, detector 115 comprises two or more array sensors.

System 4000 may include a plurality of beam steering optical elements 435 to direct excitation beams along excitation optical path from radiant generator 132 to sample 110 and/or to direct emissions along an emission optical path from sample 110 to detector 115. In the illustrated embodiment, each excitation spectral element 441 if coupled on a rotation 449. Beam steering optical elements 135, 435 may take the form of any of the embodiments discussed above regarding beam steering optical elements 135 discussed above regarding systems 1000, 2000, 3000. In the illustrated embodiment shown in FIG. 4, system 4000 comprises six beam steering optical elements 435, one for each of the six excitation spectral elements 441. One or more of the beam steering optical elements 135, 435 may comprise one or more spectrally selective optical elements (e.g., a dichroic mirror, filter, or beamsplitter) characterized by a spectrum corresponding to or complementing the spectrum of one of the radiant sources 401 and/or one of the emission spectral elements 421. In certain embodiments, one or more of the beam steering optical elements 135, 435 may comprise a neutral density filter having a constant, or essentially constant, transmission over a broad band of wavelengths (e.g., having constant transmission over the visible, infrared, and/or UV wavelength bands, over the wavelength band of some or all the spectral elements 421, 441, and/or over some or all of the wavelength band of radiant source 400). For example, one or more of the beam steering optical elements 135, 435 may comprise one or more neutral density filter having a transmission of 1%, 5%, 10%, 20%, 25%, 50%, 75%, 80%, 90%, 95%, or 99% over a given wavelength band. In the illustrated embodiment, beam steering optical elements 435 are coupled to filter wheel 449. Alternatively, beam steering optical elements 435 may be coupled to filter wheel 429 such that each beam steering optical element 435 corresponds to a respective one of the emission spectral elements 421a-f. In yet other embodiments, beam steering optical elements 135, 435 may be moved independently of radiant sources 401, excitation spectral elements 441, and emission spectral elements 421. The number of beam steering optical elements 135, 435 may be less than or greater than the total number of radiant sources 401 or the total number of emission spectral elements 421, for example, to increase the number of wavelength bands provide by the excitation spectral elements 441 or the total number of emission spectral elements 421.

Spectral elements 121 (e.g., 121a, 121b), 141 (e.g., 141a, 141b), 421 (e.g., 421a-f), 441 (e.g., 441a-f) illustrated in FIG. 1 to FIG. 4 may comprise a spectral filter, wherein each filter is characterized by a transmission wavelength band or range and/or an average transmission wavelength. Any of the spectral elements 121, 141, 421, 441 may comprise a transmissive or reflective optical filter configured to filter emissions from the sample into a desired or predetermined wavelength range or band. For example, any of the spectral elements 121, 141, 421, 441 may comprise one or more of a colored filter, a dichroic filter, a dichroic mirror, or a dichroic beamsplitter. In certain embodiment, spectral elements 121, 141, 421, 441 may comprise emission from sample 110 that have passed through a dispersive optical element, such as a prism or diffractive grating, spectrometer, or spectrophotometer. In such embodiments, each spectral element 121, 141, 421, 441 comprises a different portion of the spectrum produced by the dispersive optical element. Emission spectral elements 121, 421 may be attached to a filter wheel (e.g., filter wheel 429 in FIG. 4) or similar motion device to move different ones of elements 121, 421 into and out of an optical path between detector 115 and sample 110.

Any of radiant sources 101 (e.g., 101a and/or 101b), 401 illustrated in FIG. 1 to FIG. 4 may comprises one or more radiant generators 132 (e.g., one or more light sources or other broadband sources of light and/or UV radiation) in combination with a respective one from a plurality of excitation spectral elements 141a, 141b. Additionally or alternatively, one or more of the radiant sources 101, 401 may comprise a radiant generator 132 having a relatively narrow wavelength range or band, such as a colored or narrow band LED, laser, discharge tube, or the like, having a spectrum or wavelength band of light or other electromagnetic radiation selected to excite a dye that may be associated with a respective target molecule. In such embodiments, one or more of the excitation spectral elements 141 (e.g., 141a, 141b), 441 (e.g., any of 441a-f) may be optionally included, for example, to further narrow or otherwise condition the spectrum of radiation directed to sample 110. As illustrated in FIG. 4, excitation spectral elements 441 may be attached to a filter wheel 449 or similar motion device to move different ones of elements 441 into and out of an optical path between radiant generator 132 and sample 110. In some embodiments, radiant sources 101, 401 may each comprise a radiant generator and chromatically dispersive optical element configured to transmit or reflect radiation from the radiant generator, each radiant source including a different portion of a spectrum from the chromatically dispersive optical element.

Sample holder 112 in systems 1000, 2000, 3000, 4000 may comprise a plate or surface in which sample 110 is disposed on sample holder 112 or disposed within sample holder 112, for example, within a sample well, through-hole, or surface region. In embodiments, sample 110 comprises a plurality of spatially separated sample portions, for example, a sample solution separated into a plurality of spatially separated sample sites such as a plurality of sample wells, through-holes, or surface regions. Each sample portion may contain an identical, or substantially identical, amount of one or more target molecules, may contain different amounts or types of one or more target molecules, or contain no target molecules (e.g., dPCR sample portions). Sample holder 112 may comprise a strip or microtiter plate (e.g., a strip of 4, 6, or 8 wells or a microtiter plate comprising 24 wells, 48 well, 96 wells, 384 wells, 1536 wells, or 3456 wells). Alternatively, sample holder 112 may comprises a card or plate comprising an array of reaction sites or chambers that each contain a portion of sample 110 (e.g., a card or plate comprising 384 or 9,216 sample chambers or reaction sites). In some embodiments, sample holder 112 comprises a plate or chip including an array of spatially separated through-holes that each contain a portion of sample 110 (e.g., a through-hole plate or chip containing 3,072 through-holes, 20,000 through-holes, 50,000 through-holes, 100,000 through-holes, or more than 100,000 through-holes). In certain embodiments, sample holder 112 comprises a tube, channel, or capillary comprising a plurality of sample droplets or slugs containing a portion of sample 110 and, optionally, an immiscible fluid or oil disposed between the droplets. Each droplet or slug may contain an identical, or substantially identical, amount of one or more target molecules, may contain different amounts or types of one or more target molecules, or contain no target molecules (e.g., dPCR sample droplets or slug).

Where applicable, processor 130 and associated memory of system 4000 may incorporate any of the elements or features of the processor 130 discussed above herein regarding systems 1000, 2000, 3000. In the illustrated embodiment shown in FIG. 4, processor 130 and associated memory may be configured to comprise any of the instructions discussed above in relation to FIG. 1 to FIG. 3, for example, in performing an amplification assay, illuminating sample 110 with any of the radiant sources 401a-f, and/or detecting or measuring emissions from sample 110 with any of emission spectral elements 421a-f. In certain embodiments of systems 1000, 2000, 3000, 4000, all or portions of processor 130 and/or the associated memory may be integrated into a single instrument that may be enclosed into a housing. In some embodiments, all or portions of processor 130 and/or the associated memory may be separately located from the rest of system 1000, wherein they physically connected to one another via a physical cable, a local area network, a wide area network, or the like. Additionally or alternatively, they may be wirelessly connection to one another via a Wi-Fi connection, a Bluetooth connection, a cloud connection, or the like.

In certain embodiments, any of systems 1000, 2000, 3000, 4000 may comprise a single system or instrument having one or more processors 130 and associated memory containing instructions discussed above herein for the respective system. Alternatively, any of systems 1000, 2000, 3000, 4000 may comprise a second system or instrument configured to perform one or more of these tasks. In such embodiments, systems 1000, 2000, 3000, 4000 may comprise two or more systems or instruments that each perform some of these tasks. Each such system or instrument may have its own processor and/or memory or, alternatively, share a common processor and/or memory, for example using a centralized storage system, such as a cloud storage system, and/or a centralized processor or processor system, such as a cloud computing processor or system.

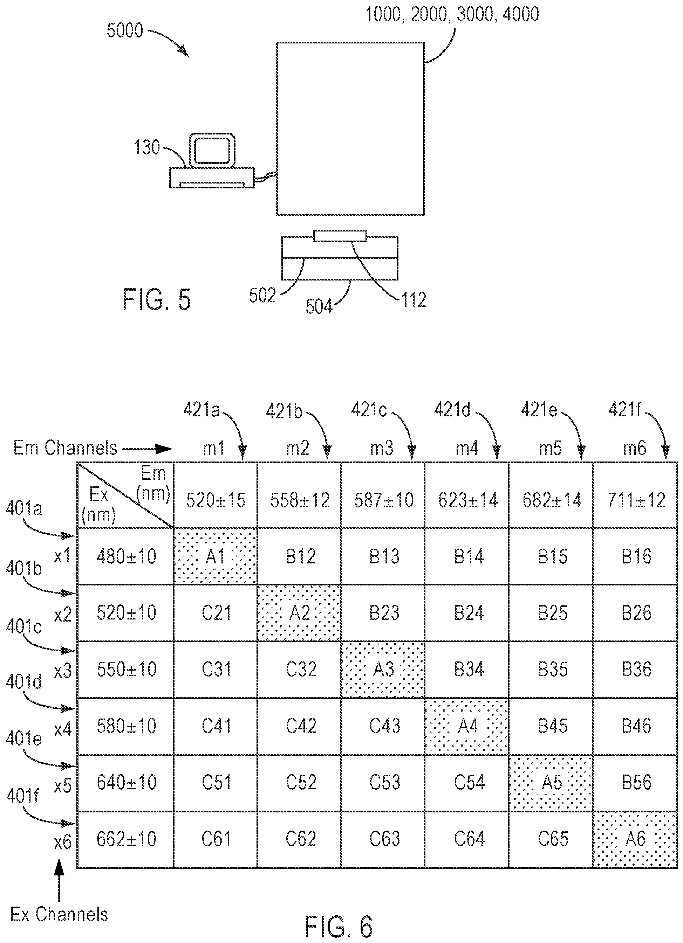

Systems 1000, 2000, 3000, 4000 may be an end-point system or instrument configured to provide measure emissions from one or more dyes in sample 110 after the conclusion of an amplification assay, such as a PCR assay (e.g., a dPCR system or instrument or a system or instrument configured to perform a melt assay). Referring to FIG. 5, in certain embodiments, an amplification system 5000, comprises the optical components of systems 1000, 2000, 3000, or 4000 (e.g., radiant sources, spectral elements, detectors, beam steering optical elements, and the like), processor 130, sample holder 112, and a thermal controller or system including a thermal block 502 configured to receive sample holder 112 and a temperature controller or thermal cycler 504 configured to perform a PCR or other amplification assay. System 5000 may further include a heated cover (not shown) that is located opposite thermal block 502 and configured to further control the temperature and/or environment of sample holder 112 and/or sample 110. Processor 130 is configured to control system 5000 during performance of the amplification assay and to operate the optical components to detect or measure emissions from one or more dyes present in sample 100 one or more times during the amplification assay. System 5000 may be further configured to detect or measure emissions from one or more dyes after completion of the amplification assay, for example, for use in determining an amount of one or more molecules associated with the one or more dyes or for detecting or measuring emissions from the dyes during a melt assay performed after the amplification assay. In certain embodiments, system 5000 performs an amplification assay on sample 110 without the use of thermal cycling. In such embodiment, system 5000 may be configured without thermal block 502 and/or without thermal cycler 504.

Referring to FIG. 6, an example is given for a selection of radiant sources 401 (e.g., radiant generator 132 in combination with excitation spectral elements 441a-f) and emission spectral elements 421 in accordance with an embodiment of system 4000 and/or 5000. Each entry in the column labeled "Ex" is an excitation "channel" of system 4000, where each Ex or excitation channel indicates wavelength range or band for illumination of sample 110. The number shown for each excitation channel is an average or central excitation wavelength for a respective one of radiant sources 401a-f. The wavelength band for each excitation channel is indicated by a "±" numerical value in nanometers about the average or central transmission wavelength for that excitation or Ex channel (e.g., the wavelength band of excitation or Ex channel x1 in the illustrated embodiment is 480±10 nanometers, or 470 nanometers to 490 nanometers). In certain embodiments, the selection of the average or central excitation wavelength may be vary from that shown in FIG. 6, for example, to accommodate a particular set of dyes, selection of target molecules, and/or assay chemistry. For example, the average or central wavelength for Ex channels x1-x6 may select to be within a range of ±5 nanometers about the values shown in the table of FIG. 6 (e.g., the average or central wavelength for Ex channel x1 may select to be 480 nanometers ±5 nanometers, the average or central wavelength for Ex channel x2 may select to be 520 nanometers ±5 nanometers, etc.). In addition, the width of the Ex channels may be wider or narrower than the widths indicated in FIG. 6. For example, the width for any of the Ex channels may less than or equal to ±5 nanometers, ±10 nanometers, ±12 nanometers, ±15 nanometers, ±18 nanometers, ±20 nanometers, about average or central value for a given channel.

Each entry in the row labeled "Em" is an emission "channel" of system 4000, where each Em or emission channel represents emissions (e.g., fluorescent emissions) from sample 110 that are received by detector 115 after being transmitted, reflected, and/or diffracted by a respective one of the emission spectral elements 421 (e.g., 421a-f). The number shown for each emission channel is an average or central emission wavelength for a respective one of emission spectral element 421. The wavelength band for each emission channel is indicated by a "±" numerical value in nanometers about the average or central transmission wavelength for that emission or Em channel (e.g., the wavelength band of emission or Em channel m1 in the illustrated embodiment is 520±15 nanometers, or 505 nanometers to 535 nanometers). In certain embodiments, the selection of the average or central emission wavelength may be vary from that shown in FIG. 6, for example, to accommodate a particular set of dyes, selection of target molecules, and/or assay chemistry. For example, the average or central wavelength for Em channels m1-m6 may select to be within a range of ±5 nanometers about the values shown in the table of FIG. 6 (e.g., the average or central wavelength for Em channel m1 may select to be 480 nanometers ±5 nanometers, the average or central wavelength for Em channel m2 may select to be 520 nanometers ±5 nanometers, etc.). In addition, the width of the Em channels may be wider or narrower than the widths indicated in FIG. 6. For example, the width for any of the Em channels may less than or equal to ±5 nanometers, ±10 nanometers, ±12 nanometers, ±15 nanometers, ±18 nanometers, ±20 nanometers, about average or central value for a given channel.

The remaining elements to the right of the Ex column and below the Em role in FIG. 6 represent various excitation/emission channel combinations or pairs (herein referred to as ex-em channel combinations or pairs, or simply channel combinations or pairs) suitable for detecting or measuring emissions from one or more dyes in sample 110 over a particular emission wavelength range for a particular excitation wavelength range. In certain embodiments, a channel combination may correspond to a dye with a maximum absorption (or excitation) wavelength that is within or near the wavelength band of the corresponding excitation channel of the combination and a maximum emission wavelength that is within or near the wavelength band of the emission channel of the corresponding excitation channel of the combination. For example, the x1, m1 channel combination (designated x1/m1 herein and represented by "A1") may be used to detect or measure a dye having (1) a maximum absorption wavelength that is within or nearest the 480±10 nanometer wavelength band of the x1 channel and (2) a maximum emission wavelength that is within or nearest the 520±15 nanometer band of the corresponding m1 channel. As another example, the x1, m3 channel combination (x1/m3 and represented by "B13") may be used to detect or measure any dye having (1) maximum absorption wavelength that is within or near the 480±10 nanometer band of the x1 channel and (2) a maximum emission wavelength that is within or near the 587±10 nanometer band of the corresponding m3 channel. Such dyes may be referred to by the excitation/emission channel to which they are readily detected or measured (e.g., as an "A1 dye" and a "B13 dye" for the current examples). It will be appreciated that a given dye may have an absorption and/or emission spectrum that extends outside the excitation/emission channel combination for which that dye has been designed.

The excitation/emission channel combinations labeled A1-A6 may be referred to as "on-axis channels" or "on-axis channel combinations", where dyes having a corresponding maximum excitation wavelength and maximum emission wavelength may be referred to as "on-axis dyes". All other excitation/emission channels combinations that are not on-axis channels in FIG. 6 may be referred to as "off-axis channels" or "off-axis channel combinations", where dyes having a corresponding maximum excitation wavelength and maximum emission wavelength may be referred to as "on-axis dyes". The off-axis channel combinations starting with the letter B (i.e., B12 to B56) are suitable for dyes also producing a Stokes shift, but these dyes may have a Stokes shift that is greater than dyes more suitable for use with on-axis channel combinations. Thus, off-axis dyes will generally produce a larger shift between a maximum absorption wavelength and a corresponding maximum emission wavelength (e.g., Stokes shift) than an on-axis dye. The off-axis channels starting with the letter C (i.e., C12 to C56) are suitable for so called "anti-Stokes" or "up-converting" fluorescent dyes, which are dyes that that produce shifted emissions in which the dye has a maximum emission wavelength or wavelength band that is less than their maximum absorption wavelength or wavelength band.

As used herein, an "on-axis channel combination" or "on-axis ex-em channel pair" is a pair of ex-em channels in which an average or central wavelength of the Em channel is shifted by an amount that is less than or equal to 60 nanometers from the corresponding Ex channel. Alternatively, for a set of Ex and Em channels, an "on-axis channel combination" or "on-axis ex-em channel pair" comprises a given Ex channel and the Em channel that has the smallest average or central wavelength shift from average or central wavelength of the given Ex channel. Under these definitions, an "off-axis channel combination" or "off-axis ex-em channel pair" is any pair of ex-em channels that is not an "on-axis channel combination" or "on-axis ex-em channel pair".

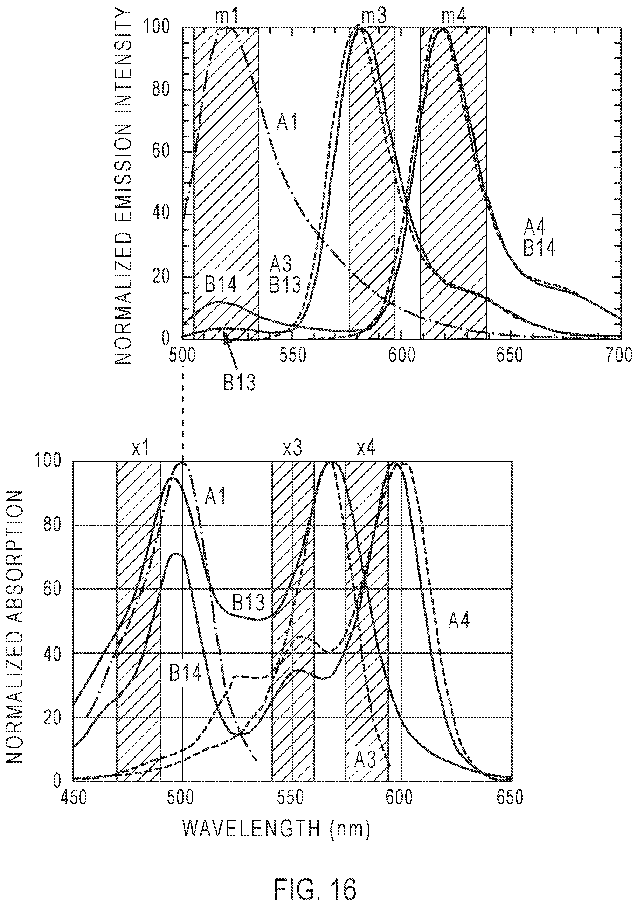

Unless otherwise noted, as used herein in the context of a system or instrument comprising one or more on-axis/off-axis channel combinations and one or more on-axis/off-axis ex-em channel combinations, an "off-axis dye" is defined as a dye having a first maximum absorption or excitation wavelength that is an absolute maximum over an entire spectrum of the dye and having a second maximum absorption or excitation wavelengths that is a local maximum and is separated from the first maximum absorption or excitation wavelength by at least 60 nanometers (e.g., see FIG. 16, in which B13 has two maximum wavelength separated by about 68 nanometers and B4 has two maximum wavelength separated by about 100 nanometers). Using this definition of an off-axis dye, an "on-axis dye" is any dye that is not an off-axis dye.

Some on-axis dyes may be a "broadened dye". As used herein, a "broadened dye" is defined as an on-axis dye having a first maximum absorption or excitation wavelength that is an absolute maximum over an entire spectrum of the dye and having a second maximum absorption or excitation wavelengths that is a local maximum and is separated from the first maximum absorption or excitation wavelength by less than 60 nanometers. A broadened dye may be used in combination with another on-axis dyes to increase the number of dyes that can be detected or measured using a combination of on-axis and off-axis channel combinations. For example, a first, on-axis dye may be selected that produces a maximum signal using an on-axis channel combination (e.g., the A1 channel combination) and used in combination with second, broadened dye that produces a higher signal in an adjacent off-axis channel combination (e.g. the B12 channel combination) than that produced by the first dye for an equal concentration of both dyes.

In certain embodiments, an "off-axis dye" is comprises a "big dye" or "energy transfer dye conjugate", where the off-axis dye comprises two "on-axis dyes": a "donor dye" and an "acceptor dye" (e.g., see US5800996) covalently attached via a linker. This definition of an off-axis dye may be irrespective of the amount of shift between any pair of local maximum absorption or excitation wavelengths. The donor dye and the acceptor dye are linked by linker, wherein the donor dye is a dye capable of absorbing light at a first wavelength to produce excitation energy and the acceptor dye is dye which is capable of absorbing at least a portion of the excitation energy produced by the donor dye and, in response, fluorescing at a second wavelength that is equal to or substantially equal to a maximum emission wavelength of the acceptor dye by itself (i.e., when not linked to the donor dye).

In certain embodiments, a pair of dyes may be considered an "on-axis dye" or an "off-axis dye" in terms of their spectral characteristic as compared to one another. For example, a first dye within a sample may be considered an "on-axis dye" and a second dye may be considered an "off-axis dye" when the first and second dyes have the same or nearly the same maximum absorption or excitation wavelength (e.g., absorption or excitation maximums that are within 5 nanometers of one another or absorb a maximum amount of radiations from a common excitation channel of an instrument or system), but a maximum emission wavelength of the second dye that is at least 50 nanometers greater than that of the first dye (e.g., referring to FIG. 16, dyes A1, B13 or B14 all have maximum absorptions wavelengths that are nearly the same, but "off-axis dyes" B13, B14 each have maximum emission wavelengths that are more than 50 nanometers greater than "on-axis dye" A1). Additionally or alternatively, a first dye within a sample may be considered an "on-axis dye" and a second dye may be considered an "off-axis dye" when the first and second dyes have the same or nearly the same maximum emission wavelengths (e.g., emission maximums that are within 2 nanometers of one another or produce maximum emissions over the wavelength band of a common emission channel of an instrument or system), but a maximum absorption or excitation wavelength of the second dye is at least 60 nanometers less than that of a maximum absorption or excitation wavelength of the first dye (e.g., referring to FIG. 16, for the dye pairs A3, B13 or A4, B14, each dye pair has nearly the same maximum absorptions wavelengths (~565 nanometers and ~600 nanometers, respectively), but "off-axis dyes" B13, B14 each have a maximum absorption/excitation wavelength that is at least 60 nanometers less than that of the "on-axis dye" A1).

In the case of six excitation channels and six corresponding emission channels, as shown in FIG. 6, the inventors have found that it is possible to obtain or multiplex all 21 different emission signals from a sample (i.e., for the channel combinations A1-A6 and B 12-B56). In certain embodiment, the inventors have found that it is possible to obtain or multiplex 18 to 20 different emission signals from a sample (e.g., using all channel combination except B56 and/or another channel combination in which the difference between the central wavelength of adjacent ex and/or em channels is small; e.g. a difference that is less than or equal to 30 nanometers or is less than or equal to 25 nanometers). As discussed below herein, the inventors have found at least 10 dyes (e.g., six on-axis dyes and four off-axis dyes) can be selected to determine or measure the amounts of 10 different target molecules contained in the same sample solution during a single PCR assay.

The combination of six excitation channels x1-x6 and six emission channels m1-m6 shown in FIG. 6 provides an exemplary embodiment suitable for illustrating various embodiments of the current disclosure. The central wavelengths and wavelength bands for each of the ex/em channels x1-x6 and m1-m6 may be modified to accommodate various system or assay configurations, for example, to provide improved measurements of a particular set of dyes and/or probes and target molecules being considered. The number of ex/em channels may be increased by increasing the number of emission channels and/or the number of emission channels, for example, by using 7, 8, or more excitation channels and/or by 7, 8, or more emission channels. Additionally or alternatively, excitation channels may be configured to cover a broader range of wavelengths, for example, extending into the UV, near UV, infrared, or near-infrared wavelength bands of the electromagnetic spectrum (e.g., including an excitation channel with a central wavelength is less than or equal to 450 or 500 nanometers, or including an excitation channel with a central wavelength that is greater than or equal to 720 or 750 nanometer). Additionally or alternatively, emission channels may be configured to cover a broader range of wavelengths, for example, extending into the UV, near UV, infrared, or near-infrared wavelength bands of the electromagnetic spectrum (e.g., including an excitation channel with a central wavelength is less than or equal to 400 or 450 nanometers, or including an excitation channel with a central wavelength that is greater than or equal to 670 or 700 nanometer). In certain embodiments, as compared to the emission channels shown in FIG. 6, the number of emission channels may be increased and/or the spectral distance between adjacent emission channels may be decreased through the use of a spectrally dispersive optical element such as a prism, diffractive grating, spectrometer, or spectrophotometer. In certain embodiments, as compared to the excitation channels shown in FIG. 6 the number of excitation channels may be increased and/or the spectral distance between adjacent excitation channels may be decreased through the use of a spectrally dispersive optical element such as a prism or diffractive grating.

The inventors have identified various combinations of dyes for which it is possible to detect and/or quantify amounts of one or more off-axis dye and, as a consequence, detect and/or quantify amounts target nucleic acids associated with respective ones of the on-axis / off-axis dyes. Various methods and dye combinations including at least one off-axis dye will now be discussed for identifying three dyes, five dyes, and ten dyes in a sample or in each sample of an array of samples.

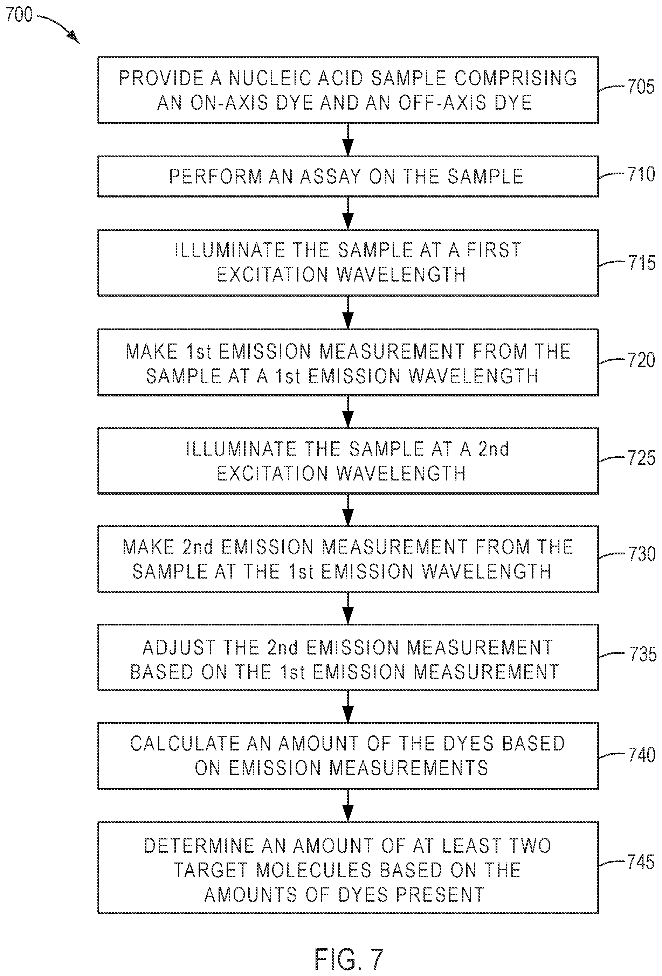

Referring to FIG. 7, in certain embodiments, a method 700 includes an element 705 comprising providing sample 110 comprising a first on-axis dye and a second off-axis dye. The method 700 also includes an element 710 comprising performing an assay on the sample. The method 700 also includes an element 715 comprising illuminating the sample with a first radiant source characterized by a first excitation wavelength and/or wavelength band. The method 700 also includes an element 720 comprising, in response to illumination in element 715, making a first emission measurement from the sample at a first emission wavelength and/or over a first wavelength band. The method 700 also includes an element 725 comprising illuminating the sample with a second radiant source characterized by a second excitation wavelength and/or wavelength band that is different than that of the first. The method 700 also includes an element 730 comprising, in response to illumination in element 725, making a second emission measurement from the sample at a second emission wavelength and/or over a second wavelength band. The method 700 may optionally include an element 735 comprising adjusting the second emission measurement based on the first emission measurement to provide an adjusted second emission measurement. The method 700 may optionally include an element 740 comprising calculating an amount of the first and second dyes based on at least some of the emission measurements. The method 700 may optionally include an element 745 comprising determining an amount of the two target molecules based on at least some of the emission measurements.

Systems 2000, 3000, 4000, or 5000 may be used to perform method 700, in which case the radiant sources may be any of those discussed herein with regard to radiant sources 101, 401, for example, radiant generator 132 in combination with excitation spectral elements 141 or 441. The first and second emission measurements from the sample may be made using one or more detectors 115 in combination with emission spectral elements 121 or 421. Referring to element 710, the assay may be a PCR assay such as a qPCR assay, a dPCR assay, a post PCR assay such as melt curve analysis, or the like.

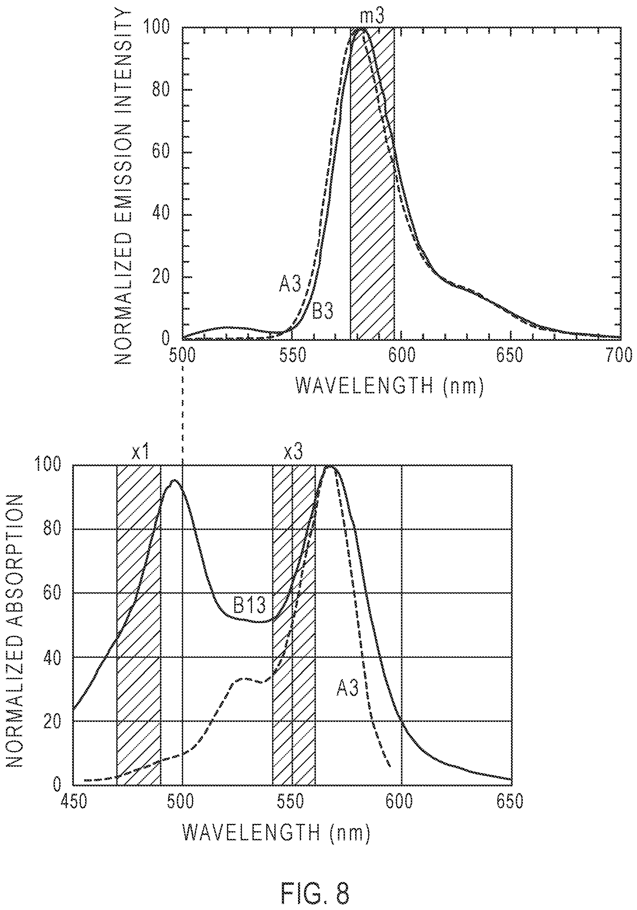

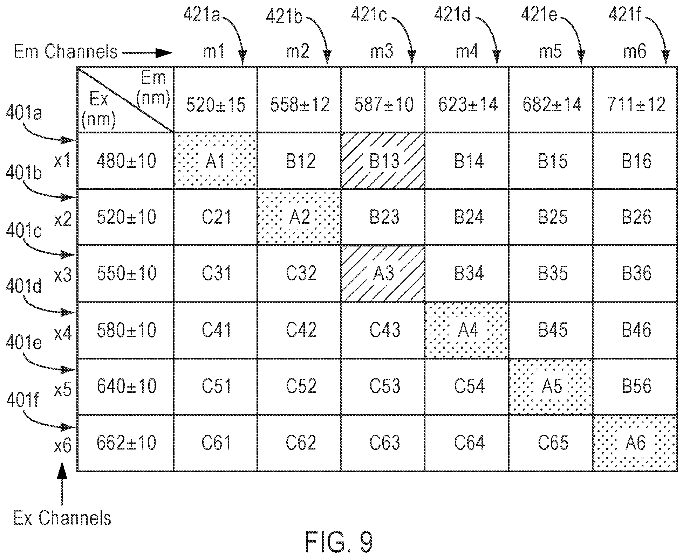

FIG. 8 shows the normalized absorption or excitation spectrum (lower plot) and the normalized emission spectra (upper plot) of two dyes suitable for use with method 700 (e.g., dyes A3 and B13). The features discussed here regarding plots in FIG. 8 are also generally applicable to the other such plots for other dyes discussed herein. The plots in FIG. 8 show normalized data for an off-axis dye referred to as B13 and an on-axis dye referred to as A3. The wavelength bands for excitation channels x1, x3 and emission channel m1, m3 from FIG. 6 are also indicated in the grayed-out area in the two plots. FIG. 9 shows the dyes A3 and B13 within the ex-em channel pair grid introduced in FIG. 6. The ex-em channel combination associated with these dyes correspond to absorption/excitation maximums and emission maximums for these two dyes. FIG. 9 shows ex-em channel combinations that may be correlated with the amount of each dye individually.

Using method 700 the amounts of the A3 and B13 may be determined using the excitation channels x1, x3 and emission channel m3 (i.e., channel combinations x1/m3 and x3/m3). Method 700 may also be used with other two dye combinations, such as disclosed herein or otherwise, that have a common or similar maximum emission wavelength (e.g., maximum emission wavelengths that are equal to one another, are within ±1 nanometer of one another, within ±2 nanometers of one another, within ±5 nanometers of one another, or within ±10 nanometers of one another) and with other ex-em channel combinations having spectral bandwidths that contains or is near to the maximum excitation and emission wavelengths of the two dyes. In the current example, the off-axis dye B13 comprises a maximum emission wavelength that is equal to or substantially equal to the maximum emission wavelength of the on-axis dye A3. In some embodiments, the dyes may comprise maximum emission wavelengths that are within 2 nanometers of one another, within 5 nanometers of one another, within 10 nanometers of one another, or within 15 nanometers of one another. In embodiments with more complex sample mixtures having more than two dyes, the selection of the emission channel m3 is selected such that the integrated energy of either or both of the two target dyes over the m3 channel bandwidth is greater than that of any other dyes within the sample when illuminated by the x1 channel and/or x3 channel.

Referring to the nominal absorption plot shown in FIG. 8, the on-axis dye is seen to have a maximum excitation wavelength that is near the x3 excitation channel spectral bandwidth. However, the off-axis dye B13 is seen to have two local maximum excitation wavelengths, one that is near the bandwidth of excitation channel x1 and another that is near the bandwidth of excitation channel x3. Alternatively, the x1 and/or x3 channel bandwidths may be selected such that any or all of the maximum excitation wavelengths of the two dyes are within the spectral bandwidth of the x1 and/or x3 excitation channels. In embodiments with more complex sample mixtures having more than two dyes, the bandwidth of the excitation channel is selected such that integrated energy of the on-axis and/or off-axis dyes over the corresponding ex channels is greater than that for other dyes contained in the same sample.

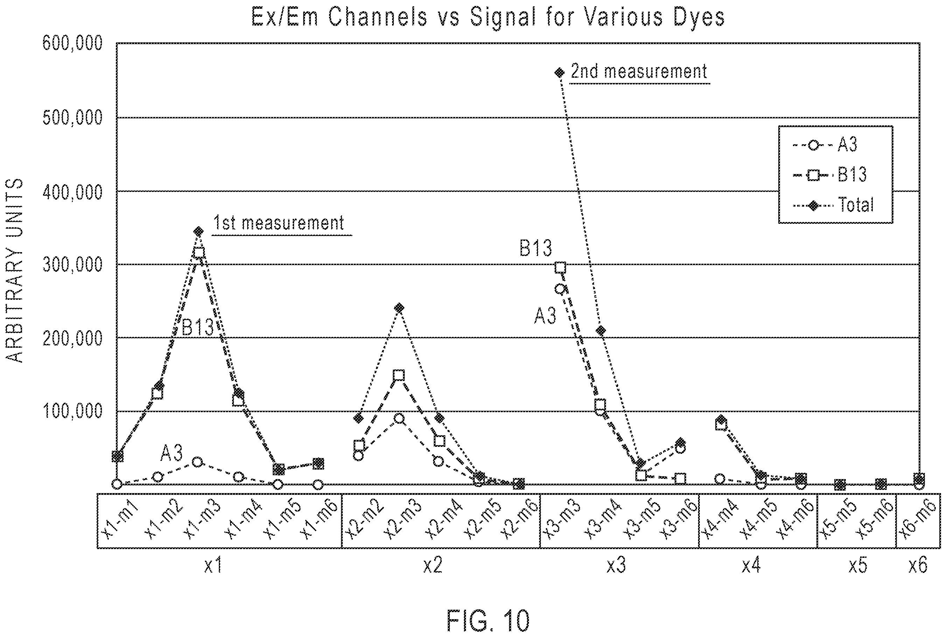

FIG. 10 shows the "ex-em channel space" or "ex-em filter space" (herein referred to as the "ex-em space") in the current example of method 700 and is based on the spectral characteristic of the dyes shown in FIG. 8 and the selected excitation and emission bands for each channel shown in FIG. 9 (i.e., for channels x1-x6 and m1-m6). FIG. 10 also shows the summation of the signals from the individual dyes at each ex-em channel combination, which is labeled as "Total". The lines between each of the data points in the plots are for clarity purposes in order to make it easier to see how the signal changes from one ex-em channel combination to the next (e.g., from x1-m1 to x1-m2, from x1-m2 to x1-m3, etc.). As can be seen, there is a break in the lines between different excitation channels (e.g., between x1-m6 and x2m2, between x2-m6 and x3-m3, etc.) so that each set of excitation channel can be distinguished from the next. As can be seen, each dye has a unique and distinctive signature, fingerprint, or pattern in the ex-em space, which the inventors have discover allows an off-axis dye to be distinguished from an on-axis dye and/or enables correction of signal interference or cross-talk between dyes when multiple dyes simultaneously produce signals that are above an ambient noise or threshold level.

The illustrated ex-em space for the A1 and B13 dyes in the current example are for a sample containing equivalent amounts of these dyes and using an instrument configured like system 4000 and the selected ex-em channel bandwidths shown. The detected signals for the first and second emission measurements in method 700 are values labeled "Total" in FIG. 10. The data in FIG. 10 for A1 and B13 are based on known amounts of these dyes in the sample for this example and on the known, distinctive dye signature, fingerprint, or pattern in the ex-em space for each dye. In general, the amount of some or all of the individual dyes is unknown and the amount of each dye and associated target molecules is determined using the method 700 or other deconvolution methods based on the first and second emission measurements in method 700.

Referring to elements 720, 730, 735 and with reference to FIG. 10, the contribution to the total signal in the second emission measurement (channel combination x3-m3) includes significant emission signals from both the off-axis dye, B13, and the on-axis dye, A3; however, the first emission measurement (channel combination x1-m3) includes emission signals almost entirely from the off-axis dye, B13. Therefore, the first emission measurement correlates well with the amount of B13 dye contained in the sample and provides a good estimate of the amount of B13 present in the sample. Based on the estimated amount of B13 from the first emission measurement, the contribution of dye B13 to the second measurement (total x3-m3 signal) can be estimated, since the spectral characteristic of the dyes are known from the ex-em data shown in FIG. 8. Thus, an adjusted second measurement can be calculated by subtracting the estimated emission of dye B13 in x3-m3 from the second measurement. The adjusted second emission measurement, therefore, correlates to the amount of A3 dye in the sample, since the contribution of the emission signal from B13 the second signal has been removed.

In certain embodiments, the adjusted second measurement can be used to provide an adjusted first measurement, since the contribution of the dye A3 signal in the x1-m3 channel can now be approximated and subtracted from the first measurement. Further iterations can also be implemented to, for example, provide a further adjusted second measurement based on the adjusted first measurement. In other embodiments, the method 700 may be modified to incorporate a system of equations that are simultaneously solved to determine or measure amounts of the A3 and B13 dyes and the associated target molecules. In any of these embodiments of the method 700, ex-em space data (FIG. 10) from any or all of the other ex-em channel combinations may be incorporated to provide more accurate estimates of the amounts of the A3 and B13 dyes, and the associated target molecules (e.g. any or all of: measurements of m1-m6 when the sample is illuminated with x1, measurements of m2-m6 when the sample is illuminated with x2, measurements of m3-m6 when the sample is illuminated with x3, measurements of m4-m6 when the sample is illuminated with x4, measurements of m5-m6 when the sample is illuminated with x5, measurements of m6 when the sample is illuminated with x6). Solving simultaneous equations using measurements from selected or all available ex-em channel combinations increased the accuracy for determining the amounts the A3 and B13 dyes when only these two dyes are present in the sample. Solving simultaneous equations using measurements from selected or all available ex-em channel combinations can also be used to determine the amount of additional dyes when 10 or more dye are present in the sample.

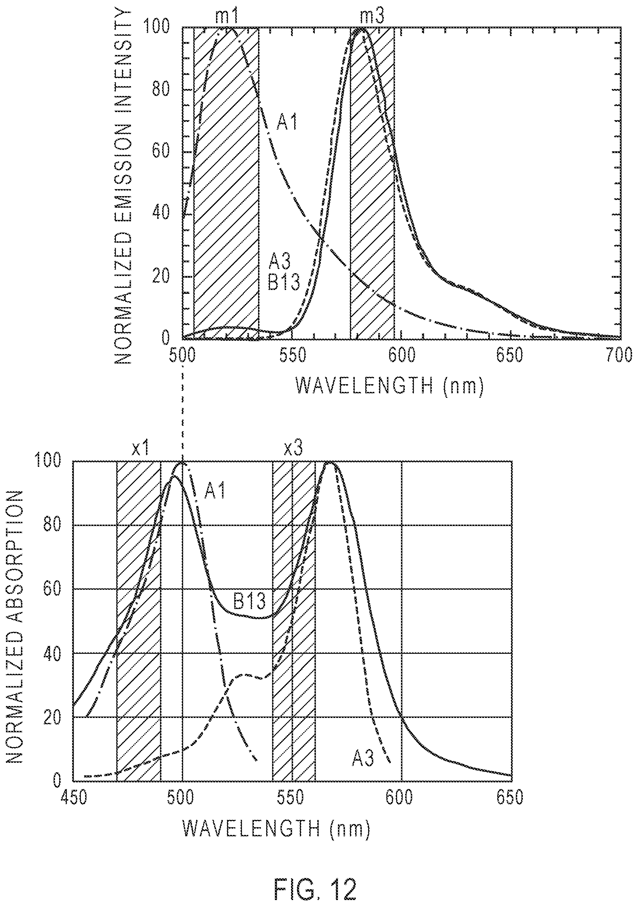

The following paragraphs explain advantages of the current teachings in terms of plots shown in FIG. 12. In prior art systems, a sample containing two or more on-axis dyes may be multiplexed to quantify an amount of the two or more corresponding target molecules to which the respective dyes are configured to bind. For example, on-axis dyes A1, A3 discussed above may both be placed in a sample containing two corresponding target molecules to which A1 and A3 are configured to bind (and may later be released to produce an emission signal). By illuminating the sample with channel x1 illumination, a large about of energy is absorbed by the A1 dye (as evidenced by the large integrated area under the absorption spectrum for A1 shown in FIG. 12 over the x1 excitation channel bandwidth). As seen in FIG. 12, most of resulting emitted energy of the A1 dye is contained in the m1 emission channel. By contrast, very little energy in the x1 channel is absorbed by the A3 dye, and most of this energy will be re-emitted in the m3 emission channel, not the m1 emission channel. Thus, the signal in emission channel m1 may be highly correlated with the amount A1 dye present in the sample. By similar review of the plots in FIG. 12, it can be seen that the combination of the x3 excitation channel with the m3 may be highly correlated with the amount A3 dye present in the sample.

However, these correlations are not exact. For example, the A1 dye has a small but measurable amount of emission in the m3 emission channel, which therefore can reduce the correlation to the A3 dye. This type of unwanted signal contribution is referred to as "cross-talk" or "signal interference". While the cross-talk is relatively small in the present example, it can be more significant when there are more than two on-axis dyes in a sample (e.g., A1, A2, and A4, or A1, A2, A3, and A4) and/or when, for a particular assay, there is a large amount of one target molecule and much less of another target molecule. These effects may be minimized by proper assay design, the use of calibration plates, and deconvolution algorithms for processing the raw emission data from each channel. The use of various off-axis channel combinations may also be used to enhance deconvolution, and thus improve the accuracy.

To compensate and/or correct for signal interference or cross-talk and other such effects, calibration plates may be used. A calibration plate contains known amounts multiple dyes. Thus, when illuminated with radiation from one or more excitation channels, the resulting fluorescent signal from each dye can be determined and the system can be corrected for cross-talk between the dyes under various illumination conditions. The calibration plate may comprise the known dye combinations in one or more reaction sites (e.g., the wells or vials of a 96 or 384 well microtiter plate), which correspond to one or more reaction sites of a test plate containing unknown amounts of a combination of target molecules. One commercially available example of a calibration plate is the Thermo Fisher calibration plate A26337. The A26337 establishes a pure dye spectra and multicomponent values for ABY^{™}, JUN^{™}, and MUSTANG PURPLE^{™} dyes on Applied Biosystems's QuantStudio^{™} 3 and 5, 96-well 0.1-mL real-time PCR systems. The formulations of the dyes in this plate improve results with multiplexing by more accurately representing the fluorescent spectra of the respective probes in your real-time PCR experiments.

The inventors have discovered that the off-axis channels may be used not only to improve deconvolution algorithms to improve the accuracy of multiple on-axis dyes, but that added information from these off-axis channels can be used to include additional, off-axis dyes into a sample, without the need to increase the number of excitation or emission channel. For example, six on-axis dyes may be identified in a sample using the six Ex channels x1-x6 and the six Em channels m1-m6 discussed herein. In order to increase the number of on-axis dyes that may be detected beyond six, additional Ex and Em channels would be needed using prior art techniques. Not only does this increase system costs and complexity, but there may be technical issues that, for example, make it difficult to increase the number of wavelength bands that can be provided utilizing optical components that are suitable for operation within the visible wavelength band.

The inventors have discovered that additional off-axis dyes may be included in sample 110 to increase the number of dyes and corresponding target molecules that can be measured with the same set of excitation and emission channels. This result was unexpected, since the introduction of additional off-axis dyes increases the problem of cross-talk discussed above in regard to multiplexing multiple on-axis dyes in the same sample. To appreciate, reference is again made to FIG. 12. For example, it can be seen that there is a large amount of cross-talk between the dyes A3 and B13, since both dyes A3 and B13 absorb significant energy in x3 excitation channel and both re-emit this energy in the m3 emission channel. Based on these observations, it would not appear possible to determine how much of the signal in the x3, m3 channel combination is from each of dyes A3, B13.

However, it has been discovered that the data from both the on-axis and off-axis channel combinations can be used to help mitigate such problems. For example, in the present case, the B13 channel combination may be used to help determine the amount dye B13 present in the sample 110. With this information, the contribution of dye B13 to the signal in the A3 channel combination may be calculated and used to modify the m3 signal to determine the amount of dye A3. This can be explained as follows. As seen in the absorption plot in FIG. 12, dye B13 absorbs a significant amount of energy over the excitation channel x1 band, while the dye A3 absorbs very little energy over excitation channel x1 band. Also, a significant amount of the x1 energy absorbed by dye B13 is emitted in the m3 channel. Thus, the x1 excitation channel can be used to determine the amount of both the A1 and B13 dyes by measuring the signals in the m1 emission channel and m3 emission channel, respectively, since dye A1 emits primarily in emission channel m1 and dye B13 emits primarily in emission channel m3 when both are illuminating using excitation channel x1. With this information for the amount of dye B13, the amount of the dye A3 may be determined using the x3 excitation channel, by adjusting the m3 emission signal for the known amount the B13 dye.

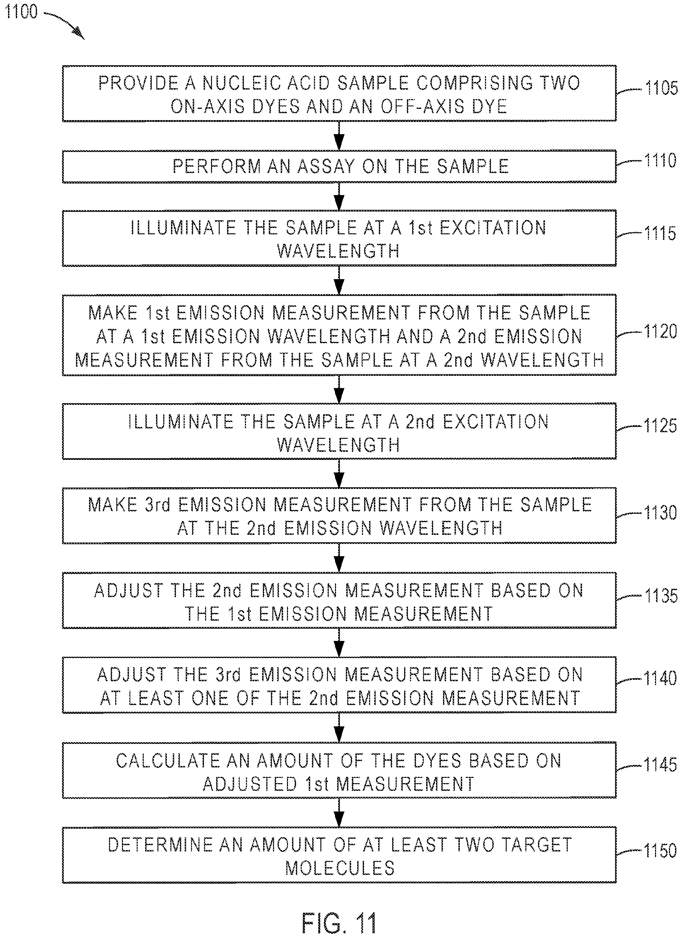

Referring to FIG. 11, in certain embodiments, a method 1100 includes an element 1105 comprising providing sample 110 comprising a first and second on-axis dye and a third off-axis dye. The method 1100 also includes an element 1110 comprising performing an assay on the sample. The method 1100 also includes an element 1115 comprising illuminating the sample with a first radiant source characterized by a first excitation wavelength and/or wavelength band. The method 1100 also includes an element 1120 comprising, in response to illumination in element 1115, making a first emission measurement from the sample at a first emission wavelength and/or over a first wavelength band and making a second emission measurement from the sample at a second emission wavelength and/or over a first wavelength band. The method 1100 also includes an element 1125 comprising illuminating the sample with a second radiant source characterized by a second excitation wavelength and/or wavelength band that is different than that of the first. The method 1100 also includes an element 1130 comprising, in response to illumination in element 1125, making a third emission measurement from the sample at the second emission wavelength and/or over the second wavelength band. The method 1100 may optionally include an element 1135 comprising adjusting the second emission measurement based on the first emission measurement to provide an adjusted second emission measurement. The method 1100 may optionally include an element 1140 comprising adjusting the third emission measurement to provide an adjusted second emission measurement that is based on at least one of the first emission measurement, the second emission measurement, and/or the adjusted second emission measurement. The method 1100 may optionally include an element 1145 comprising calculating an amount of the first, second, and third dyes based on at least some of the emission measurements. The method 1100 may optionally include an element 1145 comprising determining an amount of the three target molecules based on the amounts of the dyes present in the sample.

Systems 2000, 3000, 4000, or 5000 may be used to perform method 1100, in which case the radiant sources may be any of those discussed herein with regard to radiant sources 101 or 401, for example, radiant generator 132 in combination with excitation spectral elements 141 or 441. The emission measurements from the sample may be made using one or more detectors 115 in combination with, respectively, emission spectral elements 101a, 101b or two of the emission spectral elements 421a-f. Referring to element 1110, the assay may be a PCR assay such as a qPCR assay, a dPCR assay, a post PCR assay such as melt curve analysis, or the like. Where appropriate, any aspects of method 700 discussed above herein may also apply to method 1100, for example, regarding dyes, radiant sources, and/or emission filtering characteristics or methods of use.

FIG. 12 shows the normalized absorption spectrum and the normalized emission spectra three dyes suitable for use with method 1100 (two on-axis dyes A1, A3, and one off-axis dye B13). The features discussed here regarding plots in FIG. 12 are also generally applicable to the other such plots for other dyes discussed herein. The plots in FIG. 12 show normalized data for an off-axis dye B13 and an on-axis dyes A1 and A3. These three dyes are suitable for the ex-em channel combinations x1-m3, x1-m1 and x3-m3, respectively, shown in FIG. 6.

Using method 1100 the amounts of the A1, A3 and B13 may be determined using the excitation channels x1, x3 and emission channels x1, m3 (i.e., channel combinations x1/m3, x1/m1, and x3/m3). Method 1100 may also be used with other three dye combinations, such as disclosed herein or otherwise, where one of the on-axis dyes has a common or similar maximum emission wavelength with the off-axis dye and the other on-axis dye has a common or similar maximum excitation wavelength with the off-axis dye. Method 1100 may also be used with other ex-em channel combinations having a spectral bandwidth that contain or are near to the maximum emission and excoriation wavelengths of the three dyes. In the current example, the off-axis dye B13 comprises a maximum emission wavelength that is equal to or substantially equal to the maximum emission wavelength of the on-axis dye A3. Additionally, the off-axis dye B13 comprises a maximum excitation wavelength that is equal to or substantially equal to the maximum emission wavelength of the on-axis dye A1. In some embodiments, the dyes may comprise maximum emission wavelengths that are within 2 nanometers of one another, within 5 nanometers of one another, within 10 nanometers of one another, or within 15 nanometers of one another. In embodiments with more complex sample mixtures having more than three dyes, the selection of the emission channel m1 and m3 are selected such that the integrated energy of the three target dyes over each of the x1, x3, m1, and m3 channel bandwidths is greater than that of any other dyes within the sample when illuminated by the x1 channel and/or x3 channel and/or emitting energy in the m1 and/or m3 channels.

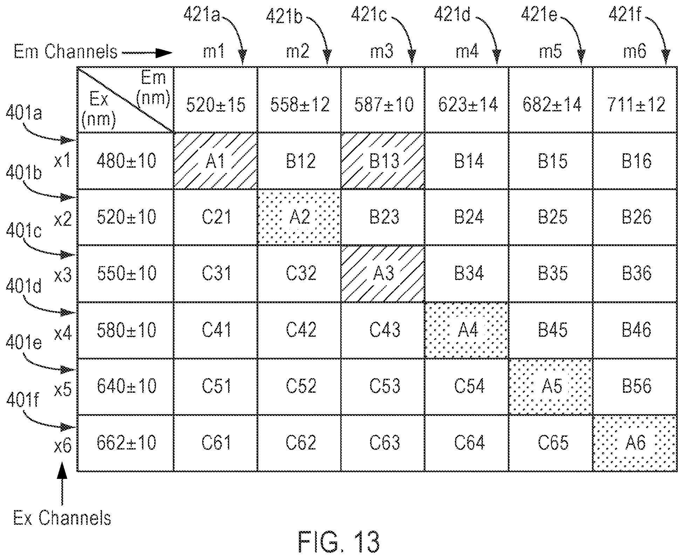

Referring to the nominal absorption plot shown in FIG. 12, the A1 on-axis dye is seen to have a maximum excitation wavelength that is near the x1 excitation channel spectral bandwidth, while the A3 on-axis dye is seen to have a maximum excitation wavelength that is near the x3 excitation channel spectral bandwidth. However, the off-axis dye B13 is seen to have two local maximum excitation wavelengths, one that is near the bandwidth of excitation channel x1 and another that is near the bandwidth of excitation channel x3. Alternatively, the x1 and/or x3 channel bandwidths may be selected such that any or all of the maximum excitation wavelengths of the three dyes are within the spectral bandwidth of the x1 and/or x3 excitation channels. In embodiments with more complex sample mixtures having more than three dyes, the bandwidth of the excitation channel is selected such that integrated energy of the on-axis and/or off-axis dyes over the corresponding ex channels is greater than that for other dyes contained in the same sample. FIG. 13 shows the dyes A1, A3, and B13 within the ex-em channel pair grid introduced in FIG. 6. The ex-em channel combination associated with these dyes correspond to absorption/excitation maximums and emission maximums for these three dyes. FIG. 13 shows ex-em channel combinations that may be correlated with the amount of each dye individually.

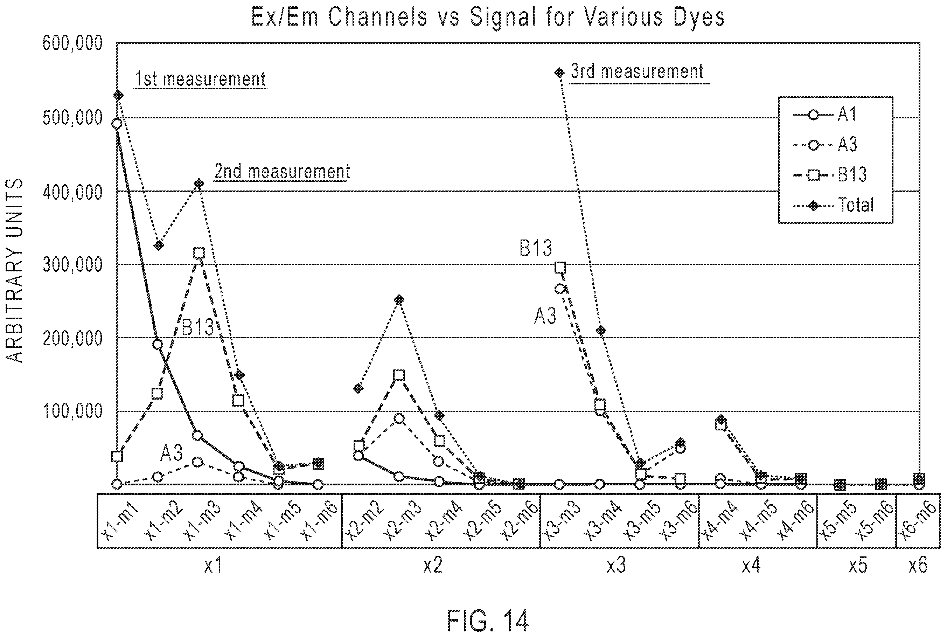

FIG. 14 shows the ex-em space for the three selected dyes used in the current example of method 1100 and is based on the spectral characteristic of the dyes shown in FIG. 12 and the selected excitation and emission bands for each channel shown in FIG. 13 (i.e., for channels x1-x6 and m1-m6). FIG. 14 also shows the summation of the signals from the individual dyes at each ex-em channel combination, which is labeled as "Total". As can be seen, each dye has a unique and distinctive signature or fingerprint in the ex-em space, which the inventors have discover allows an off-axis dye to be distinguished from an on-axis dye and/or enables correction of cross-talk between dyes when multiple dyes simultaneously produce signals that are above an ambient noise or threshold level.

The illustrated ex-em space for the A1, A3, B13 dyes in the current example are for a sample containing equivalent amounts of these dyes and using an instrument configured like system 4000 and the selected ex-em channel bandwidths shown. The detected signals for the first, second, and third measurements in method 1100 are values labeled "Total" in FIG. 14. The data in FIG. 14 for A1, A3, and B13 are based on known amounts of these dyes in the sample for this example and on the known, distinctive dye signature or fingerprint in the ex-em space for each dye. In general, the amount of some or all of the individual dyes is unknown and the amount of each dye and associated target molecules is determined using the method 1100 or other deconvolution methods based on the first, second, and third measurements in method 1100. The inventors have found that for more complex samples with greater numbers of on-axis and off-axis dyes and associated target molecules, the unique signature or fingerprint of each dye in the ex-em space, represented in FIG. 14 of this example, can be utilized to multiplex ten or more on-axis/off-axis dyes simultaneously in a common sample.

Referring to elements 1120, 1130, 1135, 1140 and to the ex-em space plots in FIG. 14, the contribution to the total signal in the second emission measurement (channel combination x1-m3) includes significant emission signals from both on-axis dyes, A1 and A3, as well as from off-axis dye, B13; however, the first emission measurement (channel combination x1-m1) includes emission signals primarily from the on-axis dye, A1. Therefore, the first emission measurement correlates well with the amount of A1 dye contained in the sample and provides a good estimate of the amount of A1 present in the sample. Based on the estimated amount of A1 from the first emission measurement, the contribution of dye A1 to the second measurement (total x1-m3 signal) can be estimated, since the spectral characteristic of the dyes are known from the ex-em data shown in FIG. 12. Thus, an adjusted second measurement can be calculated by subtracting the estimated emission of dye A1 in the x1-m3 from the second measurement. The adjusted second emission measurement, therefore, correlates to the amount of B13 dye in the sample, since the contribution of the emission signal from A3 the second signal has been removed.

In similar fashion, the contribution to the total signal in the third emission measurement (channel combination x3-m3) includes significant emission signals from off-axis dye, B13, as well as from on-axis dye, A3; however, the adjusted second emission measurement (channel combination x1-m3) provides a good estimate of the amount of B13 dye present in the sample. Based on the estimated amount of B13 from the adjusted second emission measurement, the contribution of dye B13 to the third measurement (total x3-m3 signal) can be estimated, since the spectral characteristic of the dyes are known from the ex-em data shown in FIG. 12. Thus, an adjusted third measurement can be calculated by subtracting the estimated emission of dye B13 in the x3-m3 from the third measurement. The adjusted third emission measurement, therefore, correlates to the amount of A3 dye in the sample, since the contribution of the emission signal from B13 the second signal has been removed. While the contribution of the A1 dye in the x3-m3 channel combination is insignificant in the present example, in general, method 1100 can also be used to subtract the amount of signal from the A1 dye in the x3-m3 channel combination to provide a more accurate adjusted third signal, leading to even better correlation of the adjusted third signal to the amount of the A3 dye and of the target molecule to which it is associated.

In certain embodiments, the adjusted second measurement and/or the adjusted third measurement can be used to provide an adjusted first measurement, since the contribution of the B13 dye signal and the A3 dye signal in the x1-m1 channel can now be approximated and subtracted from the first measurement (since these spectral characteristics of these dyes in any emission channel can be determined from the data in FIG. 12). Additionally or alternatively, the adjusted third measurement can be used to provide more accurate adjusted second measurement, since the contribution of the A3 dye signal in the x1-m3 channel can now be approximated and subtracted from the second measurement (since these spectral characteristics of the A3 in any emission channel can be determined from the data in FIG. 12). Further iterations can also be implemented to, for example, provide further adjusted third measurement based on the adjusted first and/or second measurements. In other embodiments, the method 1100 may be modified to incorporate a system of equations that are simultaneously solved to determine or measure the amounts of the A1, A3, and B13 dyes and the associated target molecules. In any of these embodiments of the method 1100, ex-em space data (FIG. 14) from any or all of the other ex-em channel combinations may be incorporated to provide more accurate estimates of the amounts of the A1, A3, and B13 dyes, and the associated target molecules (e.g. any or all of: measurements of m1-m6 when the sample is illuminated with x1, measurements of m2-m6 when the sample is illuminated with x2, measurements of m3-m6 when the sample is illuminated with x3, measurements of m4-m6 when the sample is illuminated with x4, measurements of m5-m6 when the sample is illuminated with x5, measurements of m6 when the sample is illuminated with x6). Solving simultaneous equations using measurements from selected or all available ex-em channel combinations increased the accuracy for determining the amounts of the A1, A3, and B13 dyes when only these three dyes are present in the sample. Solving simultaneous equations using measurements from selected or all available ex-em channel combinations can also be used to determine the amount of additional dyes when 10 or more dye are present in the sample.

Referring to element 1150, in certain embodiments, the first and second dyes are associated with and/or configured to bind or attach to different target molecules, such as first and second target polynucleotides, first and second proteins, or the like.

Referring to again to FIGS. 2-4, method 700 may be performed using any of system 3000, 4000, 5000 to measure an amount of at least a first dye and a second. Method 700 may be used to further measure or calculate an amount of a first target molecule configured to bind to the first dye and/or an amount of a second target molecule configured to bind to the second dye. In such embodiments, elements 715, 725 include illuminating sample 110 with radiant sources 101a, 101b or with first and second of radiant sources radiant sources 401a-f, where each of the two radiant sources is characterized by an average excitation wavelength that is different from that of the other (e.g., different by at least 60 nanometers). In the current embodiment, each emission spectral element 121a, 121b or two emission spectral elements 141a-f is characterized by an average emission wavelength from that is different from that of the other (e.g., different by at least 60 nanometers). In response to the illuminations, elements 720, 730 of method 700 includes:
- measuring an emission from sample 110 using detector 115 and emission spectral element 121a or a first of emission spectral elements 421 in response to illuminating the sample with radiant source 101a or the first of radiant sources 401, and
- measuring an emission from sample 110 using detector 115 and emission spectral element 121b or a second of emission spectral elements 421 in response to illuminating the sample with radiant source 101b or the second of radiant sources 401

In certain embodiments, the first dye comprises a first emission spectrum comprising a first maximum emission wavelength and the second dye comprises a second emission spectrum comprising a second maximum emission wavelength that is equal to or substantially equal first maximum emission wavelength. For example, the first dye may be an on-axis dye and the second dye may be an off-axis dye, where the maximum emission wavelength is the same or approximately the same (e.g., within 2 nanometers of one another or within 5 nanometers of one another).

Referring to again to FIGS. 3 and 4, method 1100 may be performed using any of system 3000, 4000, 5000 to measure an amount of at least a first dye, a second, and a third dye. Method 700 may be used to further measure or calculate an amount of a first target molecule configured to bind to the first dye, an amount of a second target molecule configured to bind to the second dye, and/or an amount of a third target molecule configured to bind to the third dye. In such embodiments, elements 1115, 1125 include illuminating sample 110 with radiant sources 101a, 101b or with first and second of radiant sources radiant sources 401a-f, where each of the two radiant sources is characterized by an average excitation wavelength that is different from that of the other (e.g., different by at least 60 nanometers). In the current embodiment, each emission spectral element 121a, 121b or two emission spectral elements 141a-f is characterized by an average emission wavelength from that is different from that of the other (e.g., different by at least 60 nanometers). In response to the illuminations, elements 1120, 1130 of method 1100 includes:
- measuring an emission from sample 110 using detector 115 and emission spectral element 121a or a first of emission spectral elements 421 in response to illuminating the sample with radiant source 101a or the first of radiant sources 401,
- measuring an emission from sample 110 using detector 115 and emission spectral element 121b or a second of emission spectral elements 421 in response to illuminating the sample with radiant source 101a or the first of radiant sources 401, and
- measuring an emission from sample 110 using detector 115 and emission spectral element 121b or a second of emission spectral elements 421 in response to illuminating the sample with radiant source 101b or the second of radiant sources 401
In certain embodiments:
- the first dye comprises a first absorption spectrum comprising a first maximum absorption wavelength
- the second dye comprises a second absorption spectrum comprising a second maximum absorption wavelength that is equal to or substantially equal first maximum absorption wavelength (e.g., within 2 nanometers of one another or within 5 nanometers of one another); and
- the second dye comprises a second emission spectrum comprising a second maximum emission wavelength and the third dye comprises a third emission spectrum comprising a third maximum emission wavelength that is equal to or substantially equal second maximum emission wavelength (e.g., within 2 nanometers of one another or within 5 nanometers of one another).
For example, the first and third dyes may be on-axis dyes having maximum absorption wavelengths over different excitation channels of a system or instrument.

In various embodiments, method 700 or method 1100 may further comprises performing an amplification assay on sample 110 using any of systems 1000, 2000, 3000, 4000, 5000. In such embodiments, method 700, 1100 may be performed using the various embodiments discussed herein for these systems. Embodiments of method 700, 1100 may include performing elements 715-70, 1115-1130 either during the amplification assay. For example, the amplification assay may be a real-time polymerase chain reaction (qPCR) assay in which sample 110 heated and cool over various cycles using a thermal cycler. At one or more points during one or more of the cycles, any or all of elements 715-70, 1115-1130 may be performed on sample 110. Additionally or alternatively, method 700, 1100 may be performed after the conclusion of PCR or other amplification assay. For example, after the last cycle of a PCR assay, during a melt assay after an amplification assay, or digital PCR (dPCR) assessment after an amplification assay.

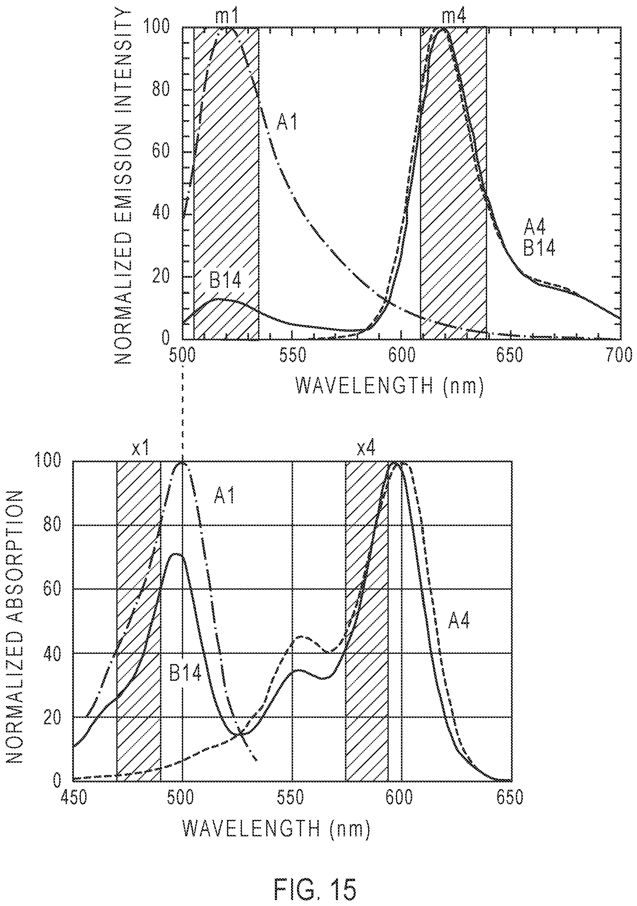

Referring to FIG. 15, the absorption or excitation spectral characteristic and the emission spectral characteristics of another triad combination of dyes is shown (dyes A1, A4, and B14). The amounts of A4 and B14 can also be determined or measured using methods 700 and/or 1100, where the same process is used as described above, but substituting channels combination x1-x4 and x4-m4 for channels combination x1-x3 and x3-m3. FIG. 16 shows the combined spectral characteristics of all five dyes discussed above (A1, A3, A4, B13, B14).

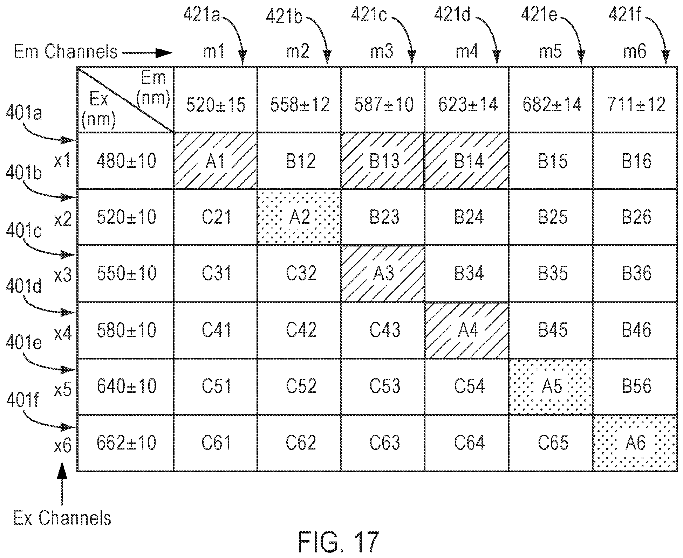

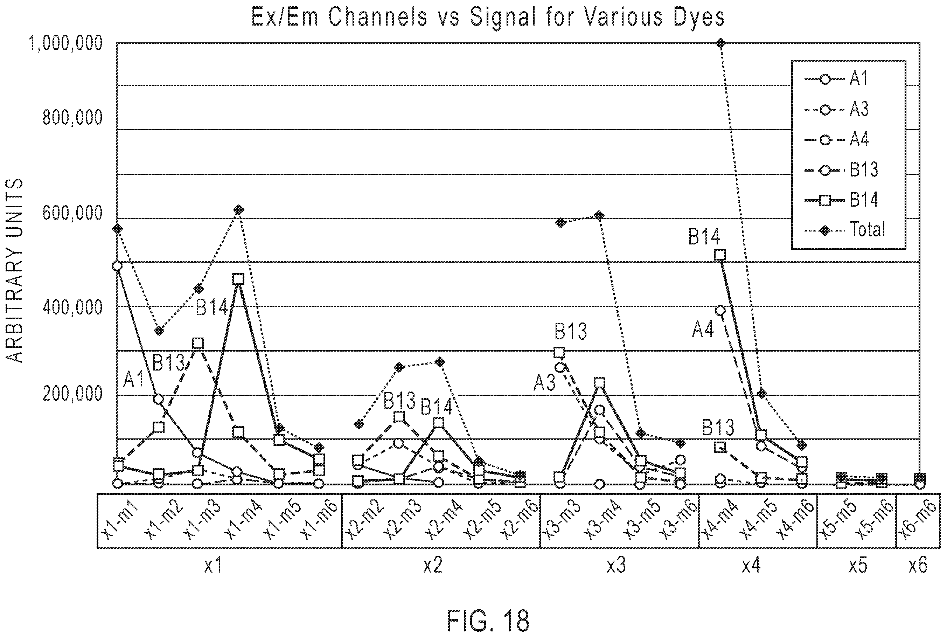

FIG. 17 shows the dyes A1, A3, A4, B13, B14 within the ex-em channel pair grid introduced in FIG. 6. The ex-em channel combination associated with these dyes correspond to absorption/excitation maximums and emission maximums for these five dyes. FIG. 17 shows ex-em channel combinations that may be correlated with the amount of each dye individually. Due to cross-talk when all five dyes are present, each dye's signature or fingerprint in the ex-em space shown in FIG. 18 may be used to reduce or eliminate the effects of crosstalk. For example, method 700, and the variations discussed above, may be used to correct for cross-talk between dyes A3, B13 and/or dyes A4, B14 contained in a sample. Additionally or alternatively, method 1100, and the variations discussed above, may be used to correct for cross-talk between dyes A1, A3, B13 and/or dyes A1, A4, B14 contained in a sample.

As seen in FIG. 18, the signatures or patterns of the A4 and B14 dyes are quite different from those of A3 and B13 dyes. For example, the contribution to the total signal from A3 and B13 is low in the x1-m4 and x4-m4 channel combinations. Therefore, in some embodiments, the A1, A4, B14 triad can be processed using method 1100 without considering the contribution of the signals from A3, B13. Alternatively, as discussed above with regard to a variation on method 1100 discussed above, a system of equations may be incorporated that are simultaneously solved determine or measure the amounts of the A1, A3, A4, B13, and B14 dyes and the associated target molecules. In certain embodiments, method 1100 may be modified to incorporate a system of equations including some or all 21 ex-em channel combinations of the "A" and "B" channel combinations shown in FIG. 6. Some or all of the ex-em space data in FIG. 18 may be incorporated to provide accurate estimates of the amounts of all five dyes and associated target molecules. In certain embodiments, the dyes A1, A3, A4, B13, B14 may various combinations of the dyes listed below in Table A. In certain embodiments, the assay performed may be a PCR assay such as a qPCR assay, a dPCR assay, a post PCR assay such as melt curve analysis, or the like.

**Table 1 - dyes for 2-plex, 3-plex, and 5-plex assays using x1, x3, x4, m1, m3, m4 channels**

| **Dye type** | **ex-em channels** | **Ex band (nm)** | **Em band (nm)** | **Suitable dyes** |
|---|---|---|---|---|
| A1 | x1-m1 | 480 ±10 | 520 ±15 | 5-FAM, 6-FAM, Oregon Green, TET, R110 |
| A3 | x3-m3 | 550 ±11 | 587 ±10 | NED, TAMRA, ABY, DY-555 |
| A4 | x4-m4 | 580 ±10 | 623 ±14 | PET, ROX, JUN, Texas Red, Alexa Fluor 594 |
| B13 | x1-m3 | 480 ±10 | 587 ±10 | FAM-TAMRA, FAM-ABY, FAM-NED |
| B14 | x1-m4 | 480 ±10 | 623 ±14 | FAM-PET, FAM-ROX, FAM-JUN, FAM-Texas Red, TET-Alexa Fluor 594 |

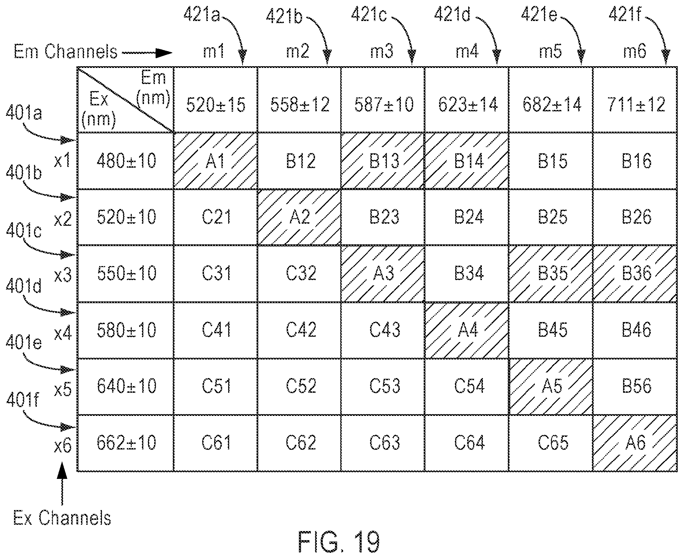

FIG. 19 shows the dyes A1-A6, B13, B14, B35, and B36 within the ex-em channel pair grid introduced in FIG. 6. The ex-em channel combination associated with these dyes correspond to absorption/excitation maximums and emission maximums for these 10 dyes. FIG. 19 shows ex-em channel combinations that may be correlated with the amount of each dye individually. The inventors have discovered various dye combinations that may be used with these ten ex-em channel combinations to permit the amount of all ten dyes and associated target molecules to be simultaneously detected and/or measured. Table 2 below shows various dyes that may be used in each of these ten ex-em combinations.

**Table 2 - dyes for 10-plex assays using six excitation and six emission channels**

| **Dye type** | **ex-em channels** | **Ex band (nm)** | **Em band (nm)** | **Suitable dyes** |
|---|---|---|---|---|
| A1 | x1-m1 | 480 ±10 | 520 ±15 | 5-FAM, 6-FAM, Oregon Green, TET, R110 |
| A2 | x2-m2 | 520 ±10 | 558 ±11 | VIC, HEX, JOE, Yakima Yellow, R6G |
| A3 | x3-m3 | 550 ±11 | 587 ±10 | NED, TAMRA, ABY, DY-555 |
| A4 | x4-m4 | 580 ±10 | 623 ±14 | PET, ROX, JUN, Texas Red, Alexa Fluor 594 |
| A5 | x5-m5 | 640 ± 10 | 682 ±14 | Alexa Fluor 647, Cy5^{®}, ATTO 647 ^{™}, DyLight 650 ^{™} |
| A6 | x6-m6 | 662 ±10 | 711 ±13 | Alexa Fluor 676, DyLight 680 ^{™} Cy5.5^{®} |
| B13 | x1-m3 | 480 ±10 | 587 ±10 | FAM-TAMRA, FAM-ABY, FAM-NED |
| B14 | x1-m4 | 480 ±10 | 623 ±14 | FAM-PET, FAM-ROX, FAM-JUN, FAM-Texas Red, TET-Alexa Fluor 594 |
| B35 | x3-m5 | 550 ±11 | 682 ±14 | ABY-Alexa Fluor 647, NED-Alexa Fluor 647, ABY-Cy5^{®}, ABY-ATTO 647 ^{™}, ABY-DyLight 650 ^{™} |
| B36 | x3-m6 | 550 ±11 | 711 ±13 | NED-Alexa Fluor 676, NED DyLight 680 ^{™}, NED-Cy5.5^{®}, ABY-Alexa Fluor 676, ABY DyLight 680 ^{™}, ABY-Cy5.5^{®} |

Comparing FIG. 19 with FIG. 17, it is seen that dyes A2, A5, A6, B35, and B36 have been added to the 5 dyes shown in FIG. 17. In certain embodiments, amounts of the on-axis dyes A2, A5, A6 in the sample may be at least be approximately determined or measured by correlation to the signal received at a detector by using on-axis, ex-em channel combinations x2-m2, x5-m5, and x6-m6. As seen in FIG. 18, the signatures or patterns of the A1, A3, A4, B13, B14 dyes have relatively low emissions in the ex-em channel combinations x2-m2, x5-m5, and x6-m6. Therefore, the cross-talk from the dyes A1, A3, A4, B13, B14 is relatively small. The amounts of the dyes A1, A3, A4, B13, B14 may be determined or measured using method 700 and/or method 1100 discussed above for these dyes. Similarly, the off-axis dyes B35 and B36, can be determined or measured with method 700 using the vertical ex-em channel combinations x5-m5 and x3-m5 for dyes B35 and A5, and ex-em using the channel combinations x6-m6 and x3-m6 for dyes B36 and A6. Additionally or alternatively, the off-axis dyes B35 and B36, can be determined or measured with method 1100 using the triad ex-em channel combinations x3-m3, x5-m5, and x3-m5 for dyes B35 and A5, and using the triad ex-em channel combinations x3-m3, x6-m6, and x3-m6 for dyes B36 and A6. Alternatively, as discussed above with regard to a variation on method 1100 discussed above, a system of equations may be incorporated that are simultaneously solved determine or measure the amounts of the A1, A2, A3, A4, A5, A6, B13, B14, B35, and B36 dyes and the associated target molecules. In certain embodiments, method 1100 may be modified to incorporate a system of equations including some or all 21 ex-em channel combinations of the "A" and "B" channel combinations shown in Table 2. Some or all of the ex-em space data in FIG. 18 may be incorporated to provide accurate estimates of the amounts of all ten dyes and their associated target molecules.

The inventors have found that for more complex samples having ten or more dyes (e.g., combinations six on-axis dyes and four off-axis dyes from Table 2) and their associated target molecules, the unique ex-em space signature or fingerprint of each dye in the ex-em space can be utilized to multiplex all the on-axis/off-axis dyes simultaneously in a common sample. FIG. 18 shows the different ex-em space signature or fingerprint of each the specific dyes A1, A3, A4, B13, B14 discussed above. As seen in comparing the signature or fingerprint for these dyes, A1 has a uniquely strong signal in the x1-m1 channel combination and a signal in the x1-m2 channel combination that is approximately two-fifths the signal in x1-m3 channel combination. By contrast, A3 and A4 have almost no signal in the x1-m1 or x1-m2 channel combinations, but instead have strong signals in the x3-m3 and x4-m4 channel combinations, respectively. Dyes B13 and B14 also have strong signal in the x3-m3 and x4-m4 channel combinations, respectively, but differ from the A3 and A4 dyes in that B13 and B14 also have relatively strong signals in the x2-m3 and x2-m4 channel combinations, respectively. While not shown in FIG. 18, similarly distinct characteristics exist for the dyes A2, A5, A6, B35, and B36. It may be noted that the dyes A2, A5, A6, B35, and B36 dyes in general have high emission in emission channels m5 and m6, while, referring to again to FIG. 18, the dyes A1, A3, A4, B13, and B14 have very little to essentially no emissions in the m5 or m6 emission channels, especially when excited by the x5 or x6 channels.

The inventors have found that for assays including both on-axis and off-axis dyes (e.g., the 8 dyes shown in FIG. 17 or the 10 dyes shown in FIG. 19), standard calibration plates, such the Thermo Fisher calibration plate A26337 discussed above herein, do not adequately correct for signal interference or cross-talk between the various dye and thus reduce the accuracy in determining the amount of each dye and a corresponding target molecule in a sample. To increase the accuracy of such determinations, the inventors have discovered that known amounts of one or more off-axis dyes should be included a calibration plate used during calibration of an instrument. For example, in embodiments using 10 dyes in a common sample or sample solution, as illustrated in FIG. 19, the inventors have found that use of a calibration plate containing four on-axis dyes plus two off-axis dyes or two on-axis dyes plus four off-axis dyes can improve the accuracy in determining or measuring the amount of each of the 10 dyes and/or the corresponding target molecules to which they are associated. For example, a calibration plate comprising FAM and VIC plus four off-axis dyes have been found to improve the accuracy determining or measuring the amount of each of the 10 dyes and/or the corresponding target molecules to which they are associated. The four off-axis dyes may include:
- one of FAM-TAMRA, FAM-ABY, or FAM-NED, and
- one of FAM-PET, FAM-ROX, FAM-JUN, FAM-Texas Red, or TET-Alexa Fluor 594, and
- one of ABY-Alexa Fluor 647, NED-Alexa Fluor 647, ABY-Cy5^{®}, ABY-ATTO 647 TM, or ABY-DyLight 650 TM, and
- one of NED-Alexa Fluor 676, NED DyLight 680 TM, NED-Cy5.5^{®}, ABY-Alexa Fluor 676, ABY-DyLight 680 TM, or ABY-Cy5.5^{®}.

Systems 4000 and/or 5000 may be used to perform methods discussed here for 10-plex sample assays of conducting a multiplex assay on a sample containing three on-axis dyes and two off-axis dyes and to analyze the resulting data to simultaneously determine or measure the amounts of ten or more dyes and their associated target molecules. In certain embodiments, such 10-plex or higher-plex assays comprise a PCR assay such as a qPCR assay, a dPCR assay, a post PCR assay such as melt curve analysis, or the like.

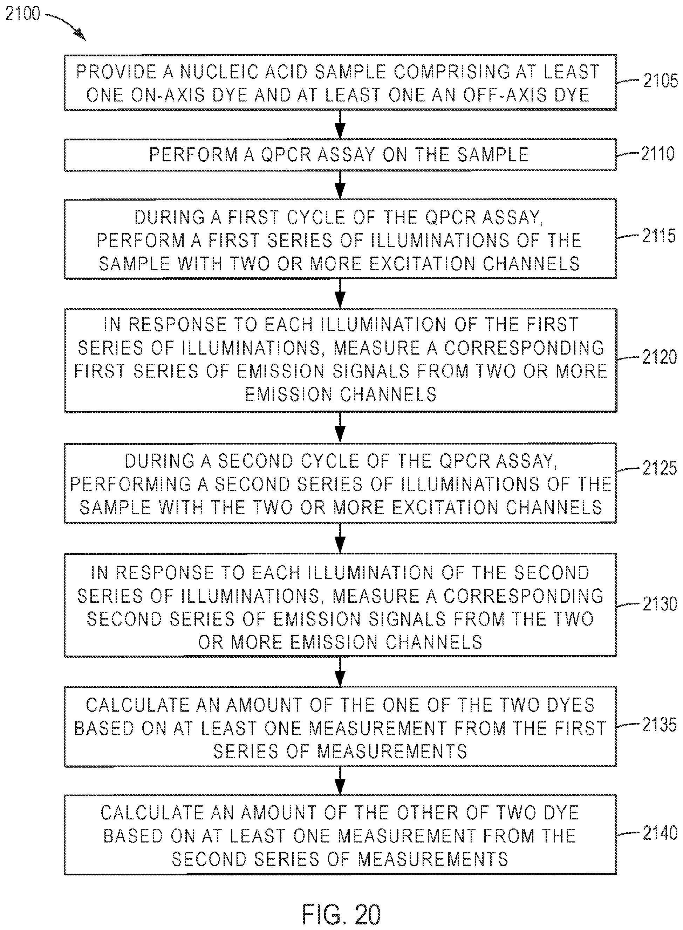

Referring to FIG. 20, in certain embodiment, systems 2000, 3000, 4000, 5000 may be used with a method 2100 of conducting or performing an amplification assay, such as a qPCR assay. Method 2100 includes an element 2105 comprising providing an off-axis dye and an on-axis dyes. Method 2100 also includes an element 2110 comprising performing an amplification assay on the sample. Method 2100 also includes an element 2115 comprising, during a first cycle of the of the amplification assay, performing a first series of illuminations of the sample with two or more excitation channels. Method 2100 also includes an element 2120 comprising, in response to each illumination of the first series of illuminations, measuring a corresponding first series of emission signals from the two or more emission channels. Method 2100 also includes an element 2125 comprising, during a second cycle of the of the amplification assay, performing a second series of illuminations of the sample with the two or more excitation channels. Method 2100 also includes an element 2130 comprising, in response to each illumination of the second series of illuminations, measuring a corresponding second series of emission signals from the two or more emission channels. Method 2100 also includes an element 2135 comprising, calculating an amount of the off-axis dye or the on-axis dye based on at least one of the measurements from the first series of measurements. Method 2100 also includes an element 2140 comprising, calculating an amount of the other of the off-axis dye and the on-axis dye based on at least one of the measurements from the second series of measurements.

The inventors have discovered that the unique signature or fingerprint of off-axis dyes, as discussed above herein, may be used increase the accuracy of measuring or calculating amounts of various dyes in a sample and/or their associated target molecules in assays in which one or more dyes produce a fluorescence signal exceeding a background fluorescence in fewer cycles than other dyes within the same sample (e.g., where one or more dyes have a lower threshold cycle, Ct, or crossing point, Cp). It will be recalled that the ex-em space plots shown in FIGS. 10, 14, and 18 represent data from samples containing equal amounts or concentrations of each dye. In some assays, a sample contains target molecules of varying number or concentration. Thus, during a qPCR or related amplification assay, dyes associated with target molecules of greater number or concentration, the signals from these dyes will be detected during earlier cycles in the amplification assay and, therefore, prior to any detectable signal being generated from other dyes, so that these less abundant dyes produce no cross-talk when measuring fluorescence signals from the more abundant target molecules.

For example, referring to FIG. 18, if the one or both off-axis dyes B13 and/or B14 are associated with target molecules that are much more abundant than those associated with the on-axis dyes A3 and A4, then during early cycles within an amplification assay the detected signal in the with be primarily from the B13 dye in the x3-m3 channel combination and/or from the B14 dye in the x4-m4 channel combination. Therefore, the signal produced by the x3-m3 and/or x4-m4 channel combinations will be predominately due to the signal generated by the B13 and/or B14 dyes, respectively, which allow calculating an amount of B 13 and/or B14 present in the solution during the early cycles. Based on this calculated value of B13 and/or B 14, the amount of these dyes present during later amplification cycles may also be calculated during later cycles, since the performance of the associated target molecule based on, for example, an estimated value of Ct or Cp. Thus, once the molecules associated with A3 and/or A4 begin to fluoresce during subsequent cycles in the amplification assay, a calculated signal from B13 and/or B14 may be subtracted from the detected signal from x3-m3 and/or x4-m4. The adjusted signal(s) may be used to calculate an amount of the molecules associated with the A3 and/or A4 dyes. In similar fashion, the contribution of the B13 and/or B14 in one or more of the other ex-em channel combinations may be used to more accurately calculate the amount of A3 and/or A4 in later amplification cycles.

The above presents a description of the best mode contemplated of carrying out the present disclosure, and of the manner and process of making and using it, in such full, clear, concise, and exact terms as to enable any person skilled in the art to which it pertains to make and use this disclosure. Embodiments of the present disclosure are, however, susceptible to modifications and alternate constructions from that discussed above which are fully equivalent. Consequently, it is not the intention to limit this disclosure to the particular embodiments disclosed. On the contrary, the intention is to cover modifications and alternate constructions coming within the scope of the disclosure as generally expressed by the following claims, which particularly point out and distinctly claim the subject matter of the present disclosure.

## Claims

1. A system (1000), comprising:
a radiant source (101a) **characterized by** an average excitation wavelength;
a sample (110) disposed to receive radiation from the radiant source, the sample comprising:
a first dye;
a second dye; and
a detector (115) configured to measure emissions from the sample;
a first emission spectral element (121a)
**characterized by** a first average emission wavelength;
a second emission spectral element (121b)
**characterized by** a second average emission
wavelength that is different than the first average emission wavelength; at least one processor (130)
comprising at least one memory including instructions to:
illuminate the sample with the radiant source and, in response, (1) measure emissions from the sample using the detector and the first emission spectral element and (2) measure emissions from the sample using the detector and the second emission spectral element,
wherein at least one of the dyes is an off-axis dye, an "off-axis dye" being a dye having a first maximum absorption or excitation wavelength that is an absolute maximum over an entire spectrum of the dye and having a second maximum absorption or excitation wavelengths that is a local maximum and is separated from the first maximum absorption or excitation wavelength by at least 60 nanometers,
wherein the radiant source (101a) is **characterized by** electromagnetic radiation within the visible light range, near infrared range, infrared range, or ultraviolet range.

2. The system of claim 1, wherein the first dye is an on-axis dye and the second dye is an off-axis dye, wherein an "on-axis dye" is any dye that is not an off-axis dye.

3. The system of claim 1, wherein:
the average excitation wavelength of the first radiant source is 480 ±5 nanometers and/or the first radiant source is **characterized by** a wavelength band that is less than or equal to ±12 nanometers about the average excitation wavelength, the first average emission wavelength of the first emission spectral element is 520 ±5 nanometers and/or the first emission spectral element is **characterized by** a wavelength band that is less than or equal to ±20 nanometers about the first average emission wavelength, and the second average emission wavelength of the second emission spectral element is 587 ±5 nanometers and/or the second emission spectral element is **characterized by** a wavelength band that is less than or equal to ±12 nanometers about the second average emission wavelength;
the average excitation wavelength of the first radiant source is 480 ±5 nanometers and/or the first radiant source is **characterized by** a wavelength band that is less than or equal to ±12 nanometers about the average excitation wavelength, the first average emission wavelength of the first emission spectral element is 520 ±5 nanometers and/or the first emission spectral element is **characterized by** a wavelength band that is less than or equal to ±18 nanometers about the first average emission wavelength; and the second average emission wavelength of the second emission spectral element is 623 ±5 nanometers and/or the second emission spectral element is **characterized by** a wavelength band that is less than or equal to ±18 nanometers about the second average emission wavelength;
or
the average excitation wavelength of the first radiant source is 550 ±5 nanometers and/or the first radiant source is **characterized by** a wavelength band that is less than or equal to ±14 nanometers about the average excitation wavelength, the first average emission wavelength of the first emission spectral element is 587 ±5 nanometers and/or the first emission spectral element is **characterized by** a wavelength band that is less than or equal to ±12 nanometers about the first average emission wavelength, and the second average emission wavelength of the second emission spectral element is 682 ±5 or 711 ±5 nanometers and/or the second emission spectral element is **characterized by** a wavelength band that is less than or equal to ±16 nanometers about the second average emission wavelength.

4. A system (1000, 2000, 3000), comprising:
a first radiant source (101a) **characterized by** a first average excitation wavelength;
a second radiant source (101b)
**characterized by** a second average excitation wavelength that is different than the first average excitation wavelength;
nucleic acid sample (110) disposed to receive radiation from the radiant sources, the sample comprising:
a first dye configured to bind to a first target molecule and
a second dye configured to bind to a second target molecule; and
a detector (115) configured to measure emissions from the sample;
an emission spectral element (121a)
**characterized by** an average emission wavelength:
at least one processor (130) comprising at least one memory including instructions to:
illuminate the sample with the first radiant source and, in response, measure emissions from the sample using the detector and the emission spectral element;
illuminate the sample with the second radiant source and, in response, measure emissions from the sample using the detector and the emission spectral element,
wherein at least one of the dyes is an off-axis dye, an "off-axis dye" being a dye having a first maximum absorption or excitation wavelength that is an absolute maximum over an entire spectrum of the dye and having a second maximum absorption or excitation wavelengths that is a local maximum and is separated from the first maximum absorption or excitation wavelength by at least 60 nanometers,
wherein each radiant source is **characterized by** electromagnetic radiation within the visible light range, near infrared range, infrared range, or ultraviolet range.

5. The system of claim 4, wherein one or more of the first maximum emission wavelength or second maximum emission wavelength is an absolute maximum over an entirety of the respective absorption spectrum.

6. The system of claim 4, wherein:
the first average excitation wavelength of the first radiant source is 480 ±5 nanometers and/or the first radiant source is **characterized by** a wavelength band that is less than or equal to ±12 nanometers about the first average excitation wavelength, the second average excitation wavelength of the second radiant source is 550 ±5 nanometers and/or the second radiant source is **characterized by** a wavelength band that is less than or equal to ±12 nanometers about the second average excitation wavelength, the first average emission wavelength of the first emission spectral element is 587 ±5 nanometers and/or the second emission spectral element is **characterized by** a wavelength band that is less than or equal to ±12 nanometers about the average emission wavelength;
the first average excitation wavelength of the first radiant source is 480 ±5 nanometers and/or the first radiant source is **characterized by** a wavelength band that is less than or equal to ±12 nanometers about the first average excitation wavelength, the second average excitation wavelength of the second radiant source is 580 ±5 nanometers and/or the second radiant source is **characterized by** a wavelength band that is less than or equal to ±12 nanometers about the second average excitation wavelength, the average emission wavelength of the first emission spectral element is 623 ±5 nanometers and/or the second emission spectral element is **characterized by** a wavelength band that is less than or equal to ±18 nanometers about the average emission wavelength;
the first average excitation wavelength of the first radiant source is 550 ±5 nanometers and/or the first radiant source is **characterized by** a wavelength band that is less than or equal to ±14 nanometers about the first average excitation wavelength, the second average excitation wavelength of the second radiant source is 640 ±5 nanometers and/or the second radiant source is **characterized by** a wavelength band that is less than or equal to ±12 nanometers about the second average excitation wavelength, the average emission wavelength of the first emission spectral element is 682 ±5 nanometers and/or the second emission spectral element is **characterized by** a wavelength band that is less than or equal to ±16 nanometers about the average emission wavelength;
the first average excitation wavelength of the first radiant source is 550 ±5 nanometers and/or the first radiant source is **characterized by** a wavelength band that is less than or equal to ±14 nanometers about the first average excitation wavelength, the second average excitation wavelength of the second radiant source is 662 ±5 nanometers and/or the second radiant source is **characterized by** a wavelength band that is less than or equal to ±12 nanometers about the second average excitation wavelength; and the average emission wavelength of the first emission spectral element is 711 ±5 nanometers and/or the second emission spectral element is **characterized by** a wavelength band that is less than or equal to ±16 nanometers about the average emission wavelength.

7. The system of claim 4,
wherein the nucleic acid sample further comprises a third dye configure to bind to a third target molecule;
wherein the system comprises:
a first emission spectral element **characterized by** a first average emission wavelength;
a second emission spectral element **characterized by** a second average emission wavelength that is different than the first average emission wavelength; and
wherein the at least one processor comprising at least one memory including instructions to:
illuminate the sample with the first radiant source and, in response, (1) measure emissions from the sample using the detector and the first emission spectral element and (2) measure emissions from the sample using the detector and the second emission spectral element;
illuminate the sample with the second radiant source and, in response, measure emissions from the sample using the detector and the second emission spectral element.

8. The system of claim 7, wherein one or more of the first maximum absorption wavelength, the second maximum absorption wavelength, second maximum emission wavelength, or third maximum emission wavelength, is an absolute maximum over an entirety of the respective spectrum.

9. The system of claim 1, 4 or 7, wherein the second dye is an off-axis dye.

10. The system of claim 1 or 7, wherein the at least one memory further comprises instructions to determine an amount of any target molecules present in the sample based on the measured emissions.

11. The system of claim 7, wherein each of the radiant sources is **characterized by** radiation having a maximum wavelength and/or average wavelength in the visible light spectrum or the infrared wavelength band and/or ultraviolet wavelength band.

12. The system of claim 7, wherein at least one of the radiant sources comprises a light emitting diode (LED) or a laser.

13. The system of claim 7, wherein:
the first radiant source comprises a radiant generator and a first filter are configured to filter radiation from the radiant generator; and
the second radiant source comprises the radiant generator and a second filter are configured to filter radiation from the radiant generator.

14. The system of claim 13, further comprising a filter wheel comprising the filters.

15. The system of claim 7, wherein the radiant sources each comprise a radiant generator and chromatically dispersive optical element configured to transmit or reflect radiation from the radiant generator, each radiant source including a different portion of a spectrum from the chromatically dispersive optical element.

16. The system of claim 15, wherein the radiant generator comprises a light source.

17. The system of claim 7, further comprising a filter wheel comprising the emission spectral elements, the filter wheel being configured to sequentially place the emission spectral element along an optical path between the sample and the detector in order to measure emissions from the sample.

18. A method, comprising:
providing a sample (110) comprising a first dye and a second dye; and:
(i) illuminating the sample with a radiant source (101a) and, in response, measuring an emission from the sample using a detector (115) and a first emission spectral element (121a) **characterized by** a first average emission wavelength and measuring an emission from the sample using a detector and a second emission spectral element (121b) **characterized by** a second average emission wavelength that is different than the first average emission wavelength; or
(ii) performing an amplification assay on the sample; illuminating the sample with a first radiant source (101a) **characterized by** a first average excitation wavelength and, in response, measuring an emission from the sample using a detector and a first emission spectral element **characterized by** a first average emission wavelength; and illuminating the sample with a second radiant source (101b)
**characterized by** a second average excitation wavelength that is different than the first average excitation wavelength and, in response, measuring an emission from the sample using the detector (115) and the second emission spectral element,
wherein at least one of the dyes is an off-axis dye, an "off-axis dye" being as a dye having a first maximum absorption or excitation wavelength that is an absolute maximum over an entire spectrum of the dye and having a second maximum absorption or excitation wavelengths that is a local maximum and is separated from the first maximum absorption or excitation wavelength by at least 60 nanometers and wherein each radiant source (101a, 101b) is **characterized by** electromagnetic radiation within the visible light range, near infrared range, infrared range, or ultraviolet range.

## Patentansprüche

1. System (1000), umfassend:
eine Strahlungsquelle (101a), **gekennzeichnet durch** eine mittlere Anregungswellenlänge;
eine Probe (110), die so angeordnet ist, dass sie Strahlung von der Strahlungsquelle empfängt, wobei die Probe umfasst:
einen ersten Farbstoff;
einen zweiten Farbstoff; und
einen Detektor (115), der zum Messen von Emissionen aus der Probe konfiguriert ist;
ein erstes Emissionsspektralelement (121a), **gekennzeichnet durch** eine erste mittlere Emissionswellenlänge;
ein zweites Emissionsspektralelement (121b), **gekennzeichnet durch** eine zweite mittlere Emissionswellenlänge, die sich von der ersten mittleren Emissionswellenlänge unterscheidet;
mindestens einen Prozessor (130), der mindestens einen Speicher umfasst, der Anweisungen enthält zum:
Beleuchten der Probe mit der ersten Strahlungsquelle und als Reaktion darauf, (1) Messen der Emissionen der Probe unter Verwendung des Detektors und des ersten Emissionsspektralelements und (2) Messen der Emissionen der Probe unter Verwendung des Detektors und des zweiten Emissionsspektralelements,
wobei mindestens einer der Farbstoffe ein Off-Axis-Farbstoff ist, wobei ein "Off-Axis-Farbstoff" ein Farbstoff ist, der eine erste maximale Absorption oder Anregungswellenlänge aufweist, die ein absolutes Maximum über ein gesamtes Spektrum des Farbstoffs darstellt, und eine zweite maximale Absorption oder Anregungswellenlänge aufweist, die ein lokales Maximum darstellt und von der ersten maximalen Absorption oder Anregungswellenlänge um mindestens 60 Nanometer getrennt ist,
wobei die Strahlungsquelle (101a) **gekennzeichnet ist durch** elektromagnetische Strahlung im sichtbaren Lichtbereich, im nahen Infrarotbereich, im Infrarotbereich oder im Ultraviolettbereich.

2. System nach Anspruch 1, wobei der erste Farbstoff ein On-Axis-Farbstoff und der zweite Farbstoff ein Off-Axis-Farbstoff ist, wobei ein "On-Axis-Farbstoff" jeder Farbstoff ist, der kein Off-Axis-Farbstoff ist.

3. System nach Anspruch 1, wobei:
die mittlere Anregungswellenlänge der ersten Strahlungsquelle 480 ± 5 Nanometer beträgt und/oder die erste Strahlungsquelle **gekennzeichnet ist durch** ein Wellenlängenband, das kleiner oder gleich ± 12 Nanometer um die mittlere Anregungswellenlänge ist, die erste mittlere Emissionswellenlänge des ersten Emissionsspektralelements 520 ± 5 Nanometer beträgt und/oder das erste Emissionsspektralelement **gekennzeichnet ist durch** ein Wellenlängenband, das kleiner oder gleich ± 20 Nanometer um die erste mittlere Emissionswellenlänge ist, und die zweite mittlere Emissionswellenlänge des zweiten Emissionsspektralelements 587 ± 5 Nanometer beträgt und/oder das zweite Emissionsspektralelement **gekennzeichnet ist durch** ein Wellenlängenband, das kleiner oder gleich ± 12 Nanometer um die zweite mittlere Emissionswellenlänge ist;
die mittlere Anregungswellenlänge der ersten Strahlungsquelle 480 ± 5 Nanometer beträgt und/oder die erste Strahlungsquelle **gekennzeichnet ist durch** ein Wellenlängenband, das kleiner oder gleich ± 12 Nanometer um die mittlere Anregungswellenlänge ist, die erste mittlere Emissionswellenlänge des ersten Emissionsspektralelements 520 ± 5 Nanometer beträgt und/oder das erste Emissionsspektralelement **gekennzeichnet ist durch** ein Wellenlängenband, das kleiner oder gleich ± 18 Nanometer um die erste mittlere Emissionswellenlänge ist; und die zweite mittlere Emissionswellenlänge des zweiten Emissionsspektralelements 623 ± 5 Nanometer beträgt und/oder das zweite Emissionsspektralelement **gekennzeichnet ist durch** ein Wellenlängenband, das um die zweite mittlere Emissionswellenlänge kleiner oder gleich ± 18 Nanometer ist;
oder
die mittlere Anregungswellenlänge der ersten Strahlungsquelle 550 ± 5 Nanometer beträgt und/oder die erste Strahlungsquelle **gekennzeichnet ist durch** ein Wellenlängenband, das kleiner oder gleich ± 14 Nanometer um die mittlere Anregungswellenlänge ist, die erste mittlere Emissionswellenlänge des ersten Emissionsspektralelements 587 ± 5 Nanometer beträgt und/oder das erste Emissionsspektralelement **gekennzeichnet ist durch** ein Wellenlängenband, das kleiner oder gleich ± 12 Nanometer um die erste mittlere Emissionswellenlänge ist, und die zweite mittlere Emissionswellenlänge des zweiten Emissionsspektralelements 682 ± 5 oder 711 ± 5 Nanometer beträgt und/oder das zweite Emissionsspektralelement **gekennzeichnet ist durch** ein Wellenlängenband, das kleiner oder gleich ± 16 Nanometer um die zweite mittlere Emissionswellenlänge ist.

4. System (1000, 2000, 3000), umfassend:
eine erste Strahlungsquelle (101a), **gekennzeichnet durch** eine erste mittlere Anregungswellenlänge;
eine zweite Strahlungsquelle (101b), **gekennzeichnet durch** eine zweite mittlere Anregungswellenlänge, die sich von der ersten mittleren Anregungswellenlänge unterscheidet;
eine Nukleinsäureprobe (110), die so angeordnet ist, dass sie Strahlung von den Strahlungsquellen empfängt, wobei die Probe umfasst:
einen ersten Farbstoff, der zur Bindung an ein erstes Zielmolekül konfiguriert ist, und
einen zweiten Farbstoff, der zur Bindung an ein zweites Zielmolekül konfiguriert ist; und
einen Detektor (115), der zum Messen von Emissionen aus der Probe konfiguriert ist;
ein Emissionsspektralelement (121a), **gekennzeichnet durch** eine mittlere Emissionswellenlänge;
mindestens einen Prozessor (130), der mindestens einen Speicher umfasst, der Anweisungen enthält zum:
Beleuchten der Probe mit der ersten Strahlungsquelle und als Reaktion darauf, Messen der Emissionen der Probe unter Verwendung des Detektors und des Emissionsspektralelements;
Beleuchten der Probe mit der zweiten Strahlungsquelle und als Reaktion darauf, Messen der Emissionen der Probe unter Verwendung des Detektors und des Emissionsspektralelements;
wobei mindestens einer der Farbstoffe ein Off-Axis-Farbstoff ist, wobei ein "Off-Axis-Farbstoff" ein Farbstoff ist, der eine erste maximale Absorption oder Anregungswellenlänge aufweist, die ein absolutes Maximum über ein gesamtes Spektrum des Farbstoffs darstellt, und eine zweite maximale Absorption oder Anregungswellenlänge aufweist, die ein lokales Maximum darstellt und von der ersten maximalen Absorption oder Anregungswellenlänge um mindestens 60 Nanometer getrennt ist,
wobei jede Strahlungsquelle **gekennzeichnet ist durch** elektromagnetische Strahlung im sichtbaren Lichtbereich, im nahen Infrarotbereich, im Infrarotbereich oder im Ultraviolettbereich.

5. System nach Anspruch 4, wobei eine oder mehrere der ersten maximalen Emissionswellenlängen und/oder der zweiten maximalen Emissionswellenlängen ein absolutes Maximum über die Gesamtheit des jeweiligen Absorptionsspektrums darstellen.

6. System nach Anspruch 4, wobei:
die erste mittlere Anregungswellenlänge der ersten Strahlungsquelle 480 ± 5 Nanometer beträgt und/oder die erste Strahlungsquelle **gekennzeichnet ist durch** ein Wellenlängenband, das kleiner oder gleich ± 12 Nanometer um die erste mittlere Anregungswellenlänge ist, die zweite mittlere Anregungswellenlänge der zweiten Strahlungsquelle 550 ± 5 Nanometer beträgt und/oder die zweite Strahlungsquelle **gekennzeichnet ist durch** ein Wellenlängenband, das kleiner oder gleich ± 12 Nanometer um die zweite mittlere Anregungswellenlänge ist, die erste mittlere Emissionswellenlänge des ersten Emissionsspektralelements 587 ± 5 Nanometer beträgt und/oder das zweite Emissionsspektralelement **gekennzeichnet ist durch** ein Wellenlängenband, das kleiner oder gleich ± 12 Nanometer um die mittlere Emissionswellenlänge ist;
die erste mittlere Anregungswellenlänge der ersten Strahlungsquelle 480 ± 5 Nanometer beträgt und/oder die erste Strahlungsquelle **gekennzeichnet ist durch** ein Wellenlängenband, das kleiner oder gleich ± 12 Nanometer um die erste mittlere Anregungswellenlänge ist, die zweite mittlere Anregungswellenlänge der zweiten Strahlungsquelle 580 ± 5 Nanometer beträgt und/oder die zweite Strahlungsquelle **gekennzeichnet ist durch** ein Wellenlängenband, das kleiner oder gleich ± 12 Nanometer um die zweite mittlere Anregungswellenlänge ist, die mittlere Emissionswellenlänge des ersten Emissionsspektralelements 623 ± 5 Nanometer beträgt und/oder das zweite Emissionsspektralelement **gekennzeichnet ist durch** ein Wellenlängenband, das kleiner oder gleich ± 18 Nanometer um die mittlere Emissionswellenlänge ist;
die erste mittlere Anregungswellenlänge der ersten Strahlungsquelle 550 ± 5 Nanometer beträgt und/oder die erste Strahlungsquelle **gekennzeichnet ist durch** ein Wellenlängenband, das kleiner oder gleich ± 14 Nanometer um die erste mittlere Anregungswellenlänge ist, die zweite mittlere Anregungswellenlänge der zweiten Strahlungsquelle 640 ± 5 Nanometer beträgt und/oder die zweite Strahlungsquelle **gekennzeichnet ist durch** ein Wellenlängenband, das kleiner oder gleich ± 12 Nanometer um die zweite mittlere Anregungswellenlänge ist, die mittlere Emissionswellenlänge des ersten Emissionsspektralelements 682 ± 5 Nanometer beträgt und/oder das zweite Emissionsspektralelement **gekennzeichnet ist durch** ein Wellenlängenband, das kleiner oder gleich ± 16 Nanometer um die mittlere Emissionswellenlänge ist;
die erste mittlere Anregungswellenlänge der ersten Strahlungsquelle 550 ± 5 Nanometer beträgt und/oder die erste Strahlungsquelle **gekennzeichnet ist durch** ein Wellenlängenband, das kleiner oder gleich ± 14 Nanometer um die erste mittlere Anregungswellenlänge ist, die zweite mittlere Anregungswellenlänge der zweiten Strahlungsquelle 662 ± 5 Nanometer beträgt und/oder die zweite Strahlungsquelle **gekennzeichnet ist durch** ein Wellenlängenband, das kleiner oder gleich ± 12 Nanometer um die zweite mittlere Anregungswellenlänge ist; und die mittlere Emissionswellenlänge des ersten Emissionsspektralelements 711 ± 5 Nanometer beträgt und/oder das zweite Emissionsspektralelement **gekennzeichnet ist durch** ein Wellenlängenband, das um die mittlere Emissionswellenlänge kleiner oder gleich ± 16 Nanometer ist;

7. System nach Anspruch 4,
wobei die Nukleinsäureprobe ferner einen dritten Farbstoff umfasst, der zur Bindung an ein drittes Zielmolekül konfiguriert ist;
wobei das System umfasst:
ein erstes Emissionsspektralelement, **gekennzeichnet durch** eine erste mittlere Emissionswellenlänge;
ein zweites Emissionsspektralelement, **gekennzeichnet durch** eine zweite mittlere Emissionswellenlänge, die sich von der ersten mittleren Emissionswellenlänge unterscheidet; und
wobei der mindestens eine Prozessor mindestens einen Speicher umfasst, der Anweisungen enthält zum:
Beleuchten der Probe mit der ersten Strahlungsquelle und als Reaktion darauf, (1) Messen der Emissionen der Probe unter Verwendung des Detektors und des ersten Emissionsspektralelements und (2) Messen der Emissionen der Probe unter Verwendung des Detektors und des zweiten Emissionsspektralelements;
Beleuchten der Probe mit der zweiten Strahlungsquelle und als Reaktion darauf, Messen der Emissionen der Probe unter Verwendung des Detektors und des zweiten Emissionsspektralelements.

8. System nach Anspruch 7, wobei eine oder mehrere der ersten maximalen Absorptionswellenlänge, der zweiten maximalen Absorptionswellenlänge, der zweiten maximalen Emissionswellenlänge oder der dritten maximalen Emissionswellenlänge ein absolutes Maximum über die Gesamtheit des jeweiligen Spektrums darstellen.

9. System nach Anspruch 1, 4 oder 7, wobei der zweite Farbstoff ein Off-Axis-Farbstoff ist.

10. System nach Anspruch 1 oder 7, wobei der mindestens eine Speicher ferner Anweisungen zum Bestimmen einer Menge aller in der Probe vorhandenen Zielmoleküle basierend auf den gemessenen Emissionen umfasst.

11. System nach Anspruch 7, wobei jede der Strahlungsquellen **gekennzeichnet ist durch** Strahlung mit einer maximalen Wellenlänge und/oder mittleren Wellenlänge im sichtbaren Lichtspektrum oder im Infrarot-Wellenlängenband und/oder Ultraviolett-Wellenlängenband.

12. System nach Anspruch 7, wobei mindestens eine der Strahlungsquellen eine Leuchtdiode (LED) oder einen Laser umfasst.

13. System nach Anspruch 7, wobei:
die erste Strahlungsquelle einen Strahlungsgenerator und einen ersten Filter umfasst, der so konfiguriert ist, dass er Strahlung vom Strahlungsgenerator filtert; und
die zweite Strahlungsquelle den Strahlungsgenerator und einen zweiter Filter umfasst, der so konfiguriert, dass er Strahlung vom Strahlungsgenerator filtert.

14. System nach Anspruch 13, das ferner ein Filterrad umfasst, das die Filter umfasst.

15. System nach Anspruch 7, wobei die Strahlungsquellen jeweils einen Strahlungsgenerator und ein chromatisch dispersives optisches Element umfassen, das so konfiguriert ist, dass es Strahlung vom Strahlungsgenerator durchlässt oder reflektiert, wobei jede Strahlungsquelle einen anderen Teil eines Spektrums von dem chromatisch dispersiven optischen Element umfasst.

16. System nach Anspruch 15, wobei der Strahlungsgenerator eine Lichtquelle umfasst.

17. System nach Anspruch 7, das ferner ein Filterrad umfasst, das die Emissionsspektralelemente umfasst, wobei das Filterrad so konfiguriert ist, dass es die Emissionsspektralelemente nacheinander entlang eines optischen Pfads zwischen der Probe und dem Detektor platziert, um Emissionen von der Probe zu messen.

18. Verfahren, umfassend:
Bereitstellen einer Probe (110), die einen ersten Farbstoff und einen zweiten Farbstoff umfasst; und:
(i) Beleuchten der Probe mit einer ersten Strahlungsquelle (101a) und als Reaktion darauf, Messen einer Emission von der Probe unter Verwendung eines Detektors (115) und eines ersten Emissionsspektralelements (121a), **gekennzeichnet durch** eine erste mittlere Emissionswellenlänge, und Messen einer Emission von der Probe unter Verwendung des Detektors und eines zweiten Emissionsspektralelements (121b), **gekennzeichnet durch** eine zweite mittlere Emissionswellenlänge, die sich von der ersten mittleren Emissionswellenlänge unterscheidet; oder
(ii) Durchführen eines Amplifikationstests an der Probe;
Beleuchten der Probe mit einer ersten Strahlungsquelle (101a), **gekennzeichnet durch** eine erste mittlere Anregungswellenlänge und als Reaktion darauf, Messen einer Emission von der Probe unter Verwendung eines Detektors und eines ersten Emissionsspektralelements, **gekennzeichnet durch** eine erste mittlere Emissionswellenlänge; und Beleuchten der Probe mit einer zweiten Strahlungsquelle (101b), **gekennzeichnet durch** eine zweite mittlere Anregungswellenlänge, die sich von der ersten mittleren Anregungswellenlänge unterscheidet, und als Reaktion darauf, Messen einer Emission von der Probe unter Verwendung des Detektors (115) und des zweiten Emissionsspektralelements,
wobei mindestens einer der Farbstoffe ein Off-Axis-Farbstoff ist, wobei ein "Off-Axis-Farbstoff" ein Farbstoff ist, der eine erste maximale Absorption oder Anregungswellenlänge aufweist, die ein absolutes Maximum über ein gesamtes Spektrum des Farbstoffs darstellt, und eine zweite maximale Absorption oder Anregungswellenlänge aufweist, die ein lokales Maximum darstellt und von der ersten maximalen Absorption oder Anregungswellenlänge um mindestens 60 Nanometer getrennt ist und wobei jede Strahlungsquelle (101a, 101b) **gekennzeichnet ist durch** elektromagnetische Strahlung im sichtbaren Lichtbereich, im nahen Infrarotbereich, im Infrarotbereich oder im Ultraviolettbereich.

## Revendications

1. Système (1000), comprenant :
une source rayonnante (101a) **caractérisée par** une longueur d'onde d'excitation moyenne ;
un échantillon (110) disposé pour recevoir un rayonnement de la source rayonnante, l'échantillon comprenant :
un premier colorant ;
un deuxième colorant ; et
un détecteur (115) configuré pour mesurer des émissions de l'échantillon ;
un premier élément spectral d'émission (121a) **caractérisé par** une première longueur d'onde d'émission moyenne ;
un second élément spectral d'émission (121b) **caractérisé par** une seconde longueur d'onde d'émission moyenne qui est différente de la première longueur d'onde d'émission moyenne ;
au moins un processeur (130) comprenant au moins une mémoire comportant des instructions pour :
éclairer l'échantillon avec la source rayonnante et, en réponse, (1) mesurer des émissions de l'échantillon à l'aide du détecteur et du premier élément spectral d'émission et (2) mesurer des émissions de l'échantillon à l'aide du détecteur et du second élément spectral d'émission,
dans lequel au moins l'un parmi les colorants est un colorant hors axe, un « colorant hors axe » étant un colorant ayant une première longueur d'onde d'absorption ou d'excitation maximale qui est un maximum absolu sur un ensemble du spectre du colorant et ayant une seconde longueur d'onde d'absorption ou d'excitation maximale qui est un maximum local et qui est séparée de la première longueur d'onde d'absorption ou d'excitation maximale par au moins 60 nanomètres,
dans lequel la source rayonnante (101a) est **caractérisée par** un rayonnement électromagnétique au sein de la plage de lumière visible, la plage de proche infrarouge, la plage d'infrarouge ou la plage d'ultraviolet.

2. Système selon la revendication 1, dans lequel le premier colorant est un colorant sur axe et le second colorant est un colorant hors axe, dans lequel un « colorant sur axe » est tout colorant qui n'est pas un colorant hors axe.

3. Système selon la revendication 1, dans lequel :
la longueur d'onde d'excitation moyenne de la première source rayonnante est de 480 ± 5 nanomètres et/ou la première source rayonnante est **caractérisée par** une bande de longueur d'onde qui est inférieure ou égale à ± 12 nanomètres autour de la longueur d'onde d'excitation moyenne, la première longueur d'onde d'émission moyenne du premier élément spectral d'émission est de 520 ± 5 nanomètres et/ou le premier élément spectral d'émission est **caractérisé par** une bande de longueur d'onde qui est inférieure ou égale à ± 20 nanomètres autour de la première longueur d'onde d'émission moyenne, et la seconde longueur d'onde d'émission moyenne du second élément spectral d'émission est de 587 ± 5 nanomètres et/ou le second élément spectral d'émission est **caractérisé par** une bande de longueur d'onde qui est inférieure ou égale à ± 12 nanomètres autour de la seconde longueur d'onde d'émission moyenne ;
la longueur d'onde d'excitation moyenne de la première source rayonnante est de 480 ± 5 nanomètres et/ou la première source rayonnante est **caractérisée par** une bande de longueur d'onde qui est inférieure ou égale à ± 12 nanomètres autour de la longueur d'onde d'excitation moyenne, la première longueur d'onde d'émission moyenne du premier élément spectral d'émission est de 520 ± 5 nanomètres et/ou le premier élément spectral d'émission est **caractérisé par** une bande de longueur d'onde qui est inférieure ou égale à ± 18 nanomètres autour de la première longueur d'onde d'émission moyenne ; et la seconde longueur d'onde d'émission moyenne du second élément spectral d'émission est de 623 ± 5 nanomètres et/ou le second élément spectral d'émission est **caractérisé par** une bande de longueur d'onde qui est inférieure ou égale à ± 18 nanomètres autour de la seconde longueur d'onde d'émission moyenne ;
ou
la longueur d'onde d'excitation moyenne de la première source rayonnante est de 550 ± 5 nanomètres et/ou la première source rayonnante est **caractérisée par** une bande de longueur d'onde qui est inférieure ou égale à ± 14 nanomètres autour de la longueur d'onde d'excitation moyenne, la première longueur d'onde d'émission moyenne du premier élément spectral d'émission est de 587 ± 5 nanomètres et/ou le premier élément spectral d'émission est **caractérisé par** une bande de longueur d'onde qui est inférieure ou égale à ± 12 nanomètres autour de la première longueur d'onde d'émission moyenne, et la seconde longueur d'onde d'émission moyenne du second élément spectral d'émission est de 682 ± 5 ou 711 ± 5 nanomètres et/ou le second élément spectral d'émission est **caractérisé par** une bande de longueur d'onde qui est inférieure ou égale à ± 16 nanomètres autour de la seconde longueur d'onde d'émission moyenne.

4. Système (1000, 2000, 3000), comprenant :
une première source rayonnante (101a) **caractérisée par** une première longueur d'onde d'excitation moyenne :
une seconde source rayonnante (101b) **caractérisée par** une seconde longueur d'onde d'excitation moyenne qui est différente de la première longueur d'onde d'excitation moyenne ;
un échantillon d'acide nucléique (110) disposé pour recevoir un rayonnement des sources rayonnantes, l'échantillon comprenant :
un premier colorant conçu pour se lier à une première molécule cible et
un deuxième colorant conçu pour se lier à une deuxième molécule cible ; et
un détecteur (115) configuré pour mesurer des émissions de l'échantillon ;
un élément spectral d'émission (121a) **caractérisé par** une longueur d'onde d'émission moyenne ;
au moins un processeur (130) comprenant au moins une mémoire comportant des instructions pour :
éclairer l'échantillon avec la première source rayonnante et, en réponse, mesurer des émissions de l'échantillon à l'aide du détecteur et de l'élément spectral d'émission ;
éclairer l'échantillon avec la seconde source rayonnante et, en réponse, mesurer des émissions de l'échantillon à l'aide du détecteur et de l'élément spectral d'émission,
dans lequel au moins l'un parmi les colorants est un colorant hors axe, un « colorant hors axe » étant un colorant ayant une première longueur d'onde d'absorption ou d'excitation maximale qui est un maximum absolu sur un ensemble du spectre du colorant et ayant une seconde longueur d'onde d'absorption ou d'excitation maximale qui est un maximum local et qui est séparée de la première longueur d'onde d'absorption ou d'excitation maximale par au moins 60 nanomètres,
dans lequel chaque source rayonnante est **caractérisée par** un rayonnement électromagnétique au sein de la plage de lumière visible, la plage de proche infrarouge, la plage d'infrarouge ou la plage d'ultraviolet.

5. Système selon la revendication 4, dans lequel une ou plusieurs de la première longueur d'onde d'émission maximale ou de la deuxième longueur d'onde d'émission maximale est un maximum absolu sur un ensemble du spectre d'absorption respectif.

6. Système selon la revendication 4, dans lequel :
la première longueur d'onde d'excitation moyenne de la première source rayonnante est de 480 ± 5 nanomètres et/ou la première source rayonnante est **caractérisée par** une bande de longueur d'onde qui est inférieure ou égale à ± 12 nanomètres autour de la première longueur d'onde d'excitation moyenne, la seconde longueur d'onde d'excitation moyenne de la seconde source rayonnante est de 550 ± 5 nanomètres et/ou la seconde source rayonnante est **caractérisée par** une bande de longueur d'onde qui est inférieure ou égale à ± 12 nanomètres autour de la seconde longueur d'onde d'excitation moyenne, la première longueur d'onde d'émission moyenne du premier élément spectral d'émission est de 587 ± 5 nanomètres et/ou le second élément spectral d'émission est **caractérisé par** une bande de longueur d'onde qui est inférieure ou égale à ± 12 nanomètres autour de la longueur d'onde d'émission moyenne ;
la première longueur d'onde d'excitation moyenne de la première source rayonnante est de 480 ± 5 nanomètres et/ou la première source rayonnante est **caractérisée par** une bande de longueur d'onde qui est inférieure ou égale à ± 12 nanomètres autour de la première longueur d'onde d'excitation moyenne, la seconde longueur d'onde d'excitation moyenne de la seconde source rayonnante est de 580 ± 5 nanomètres et/ou la seconde source rayonnante est **caractérisée par** une bande de longueur d'onde qui est inférieure ou égale à ± 12 nanomètres autour de la seconde longueur d'onde d'excitation moyenne, la longueur d'onde d'émission moyenne du premier élément spectral d'émission est de 623 ± 5 nanomètres et/ou le second élément spectral d'émission est **caractérisé par** une bande de longueur d'onde qui est inférieure ou égale à ± 18 nanomètres autour de la longueur d'onde d'émission moyenne ;
la première longueur d'onde d'excitation moyenne de la première source rayonnante est de 550 ± 5 nanomètres et/ou la première source rayonnante est **caractérisée par** une bande de longueur d'onde qui est inférieure ou égale à ± 14 nanomètres autour de la première longueur d'onde d'excitation moyenne, la seconde longueur d'onde d'excitation moyenne de la seconde source rayonnante est de 640 ± 5 nanomètres et/ou la seconde source rayonnante est **caractérisée par** une bande de longueur d'onde qui est inférieure ou égale à ± 12 nanomètres autour de la seconde longueur d'onde d'excitation moyenne, la longueur d'onde d'émission moyenne du premier élément spectral d'émission est de 682 ± 5 nanomètres et/ou le second élément spectral d'émission est **caractérisé par** une bande de longueur d'onde qui est inférieure ou égale à ± 16 nanomètres autour de la longueur d'onde d'émission moyenne ;
la première longueur d'onde d'excitation moyenne de la première source rayonnante est de 550 ± 5 nanomètres et/ou la première source rayonnante est **caractérisée par** une bande de longueur d'onde qui est inférieure ou égale à ± 14 nanomètres autour de la première longueur d'onde d'excitation moyenne, la seconde longueur d'onde d'excitation moyenne de la seconde source rayonnante est de 662 ± 5 nanomètres et/ou la seconde source rayonnante est **caractérisée par** une bande de longueur d'onde qui est inférieure ou égale à ± 12 nanomètres autour de la seconde longueur d'onde d'excitation moyenne ; et la longueur d'onde d'émission moyenne du premier élément spectral d'émission est de 711 ± 5 nanomètres et/ou le second élément spectral d'émission est **caractérisé par** une bande de longueur d'onde qui est inférieure ou égale à ± 16 nanomètres autour de la longueur d'onde d'émission moyenne.

7. Système selon la revendication 4,
dans lequel l'échantillon d'acide nucléique comprend en outre un troisième colorant conçu pour se lier à une troisième molécule cible ;
dans lequel le système comprend :
un premier élément spectral d'émission **caractérisé par** une première longueur d'onde d'émission moyenne ;
un second élément spectral d'émission **caractérisé par** une seconde longueur d'onde d'émission moyenne qui est différente de la première longueur d'onde d'émission moyenne ; et
dans lequel l'au moins un processeur comprenant au moins une mémoire comportant des instructions pour :
éclairer l'échantillon avec la première source rayonnante et, en réponse, (1) mesurer des émissions de l'échantillon à l'aide du détecteur et du premier élément spectral d'émission et (2) mesurer des émissions de l'échantillon à l'aide du détecteur et du second élément spectral d'émission ;
éclairer l'échantillon avec la seconde source rayonnante et, en réponse, mesurer les émissions de l'échantillon à l'aide du détecteur et du second élément spectral d'émission.

8. Système selon la revendication 7, dans lequel une ou plusieurs de la première longueur d'onde d'absorption maximale, de la seconde longueur d'onde d'absorption maximale, de la deuxième longueur d'onde d'émission maximale ou de la troisième longueur d'onde d'émission maximale est un maximum absolu sur un ensemble du spectre respectif.

9. Système selon la revendication 1, 4 ou 7, dans lequel le deuxième colorant est un colorant hors axe.

10. Système selon la revendication 1 ou 7, dans lequel l'au moins une mémoire comprend en outre des instructions pour déterminer une quantité de molécules cibles quelconques présentes dans l'échantillon sur la base des émissions mesurées.

11. Système selon la revendication 7, dans lequel chacune des sources rayonnantes est **caractérisée par un** rayonnement ayant une longueur d'onde maximale et/ou une longueur d'onde moyenne dans le spectre de lumière visible ou la bande de longueur d'onde infrarouge et/ou la bande de longueur d'onde ultraviolette.

12. Système selon la revendication 7, dans lequel au moins l'une parmi les sources rayonnantes comprend une diode électroluminescente (DEL) ou un laser.

13. Système selon la revendication 7, dans lequel :
la première source rayonnante comprend un générateur rayonnant et un premier filtre configuré pour filtrer le rayonnement provenant du générateur rayonnant ; et
la seconde source rayonnante comprend le générateur rayonnant et un second filtre configuré pour filtrer le rayonnement provenant du générateur rayonnant.

14. Système selon la revendication 13, comprenant en outre une roue filtrante comprenant les filtres.

15. Système selon la revendication 7, dans lequel les sources rayonnantes comprennent chacune un générateur rayonnant et un élément optique à dispersion chromatique configuré pour transmettre ou réfléchir le rayonnement provenant du générateur rayonnant, chaque source rayonnante comportant une partie différente d'un spectre de l'élément optique à dispersion chromatique.

16. Système selon la revendication 15, dans lequel le générateur rayonnant comprend une source lumineuse.

17. Système selon la revendication 7, comprenant en outre une roue filtrante comprenant les éléments spectraux d'émission, la roue filtrante étant configurée pour placer séquentiellement l'élément spectral d'émission le long d'un chemin optique entre l'échantillon et le détecteur afin de mesurer les émissions de l'échantillon.

18. Procédé, comprenant :
la fourniture d'un échantillon (110) comprenant un premier colorant et un deuxième colorant ; et :
(i) l'éclairage de l'échantillon avec une source rayonnante (101a) et, en réponse, la mesure d'une émission de l'échantillon à l'aide d'un détecteur (115) et d'un premier élément spectral d'émission (121a) **caractérisé par** une première longueur d'onde d'émission moyenne et la mesure d'une émission de l'échantillon à l'aide du détecteur et d'un second élément spectral d'émission (121b) **caractérisé par** une seconde longueur d'onde d'émission moyenne qui est différente de la première longueur d'onde d'émission moyenne ; ou
(ii) la réalisation d'un test d'amplification sur l'échantillon ;
l'éclairage de l'échantillon avec une première source rayonnante (101a) **caractérisée par** une première longueur d'onde d'excitation moyenne et, en réponse, la mesure d'une émission de l'échantillon à l'aide d'un détecteur et d'un premier élément spectral d'émission **caractérisé par** une première longueur d'onde d'émission moyenne ; et l'éclairage de l'échantillon avec une seconde source rayonnante (101b) **caractérisée par** une seconde longueur d'onde d'excitation moyenne qui est différente de la première longueur d'onde d'excitation moyenne et, en réponse, la mesure d'une émission de l'échantillon à l'aide du détecteur (115) et du second élément spectral d'émission,
dans lequel au moins l'un parmi les colorants est un colorant hors axe, un « colorant hors axe » étant un colorant ayant une première longueur d'onde d'absorption ou d'excitation maximale qui est un maximum absolu sur un ensemble du spectre du colorant et ayant une seconde longueur d'onde d'absorption ou d'excitation maximale qui est un maximum local et qui est séparée de la première longueur d'onde d'absorption ou d'excitation maximale par au moins 60 nanomètres et dans lequel chaque source rayonnante (101a, 101b) est **caractérisée par** un rayonnement électromagnétique au sein de la plage de lumière visible, la plage de proche infrarouge, la plage d'infrarouge ou la plage d'ultraviolet.
